# EUROPEAN PATENT SPECIFICATION

(11) **EP 1 206 742 B1**
(45) Date of publication and mention of the grant of the patent: **11.07.2007**
(21) Application number: 00955582.2
(22) Date of filing: 10.08.2000
(51) Int. Cl.: G06F 17/00, A61B 5/0484

(54) **ASSESSING NEUROLOGICAL CONDITIONS USING EVOKED RESPONSE POTENTIALS**
FESTSTELLUNG VON NEUROLOGISCHEN ZUSTÄNDEN MITTELS REIZEVOZIERTEN POTENTIALEN
METHODE ET PRODUIT DE PROGRAMME INFORMATIQUE PERMETTANT D'APPRECIER DES ETATS ET DES TRAITEMENTS NEUROLOGIQUES AU MOYEN DE POTENTIELS EVOQUES

(30) Priority: 10.08.1999 US 370988; 28.09.1999 US 407368
(43) Date of publication of application: 22.05.2002
(73) Proprietor: The Regents of the University of California, Oakland, CA 94607-5200 (US)
(72) Inventor: GRANGER, Richard, Irvine, CA 92312 (US)
(74) Representative: Cross, James Peter Archibald
(86) International application number: PCT/US2000/022441
(87) International publication number: WO 2001/010298

(56) References cited:
- EP-B- 0 487 110
- EP-B- 0 531 889
- EP-B- 0 557 831
- WO-A-01/45554
- US-A- 4 844 086
- US-A- 5 230 346
- US-A- 5 363 858
- US-A- 5 392 788
- US-A- 5 816 247

## Description

### Background of the Invention

### Statement as to Rights to Inventions. Made Under Federally-Sponsored Research and Development

Part of the work performed during development of this invention utilized U.S. Government funds. The U.S. Government has certain rights in this invention.

### Field of the Invention

The invention described herein relates to assessment of medical conditions, and more particularly to the assessment of neurological conditions through statistical methods.

### Related Art

It is well known that neurological anomalies can be reflected in the electrical activity of the brain. Such electrical activity is therefore commonly used to diagnose a variety of neurological disorders or to evaluate the treatment thereof. Electrical activity in the brain is typically captured and analyzed in the form of an electroencephalograph (EEG).

Neurological diagnosis using EEGs has a number of drawbacks, however. An EEG, when viewed as a waveform, can only be analyzed with respect to frequency and power. An EEG cannot be analyzed in the time domain given that an EEG represents brain activity uncorrelated to any particular event in time. Moreover, EEGs tend to have significant variance over multiple trials even when performed on the same individual. This is due, in part, to the tendency of patients to react to ambient stimuli while the EEGs are being taken. In addition, EEGs, as signals, tend to have a low signal to noise ratio (SNR). Once collected, EEGs are difficult to analyse because of these factors. Analysis of EEGs by medical personnel tends to be difficult and time consuming.

Because of the difficulties in performing EEG analysis, the use of evoked response potentials (ERPs) has been proposed. An ERP represents neural electrical activity that occurs as a result of a specific sensory stimulus to the patient, such as a flash of light or a tone. The electrical activity, measured as voltage (that is, potential), is therefore an evoked response to a stimulus. Like an EEG, an ERP is typically collected and analysed as a waveform. Unlike an EEG, however, an ERP can be analysed in the time domain as well as the frequency domain. ERPs also tend to be less variable than EEGs over multiple trials on a given patient. Nonetheless, as in the case of EEGs, artifacts occur in ERPs and their removal is difficult and error-prone. Noise reduction is also difficult. The use of ERPs as a diagnostic tool is thus expensive and time consuming. Attempts to automate ERP processing have not been widely successful.

Hence there is a need for a method of assessing the neurological condition of a patient, where the method obtains and processes relatively consistent, low noise, artifact-free data. Moreover, the diagnostic method must be fast, inexpensive, and reliable.

The document US-A-5392788 discloses a system for interpreting EEG signals by comparing them to normative EEG signals.

The document US-A-4844086 discloses a diagnostic method wherein brain evoked potential (EP) data is cross-correlated with comparable EP data from normal patients.

The document US-A-5230346 discloses a method of determining brain condition by applying selected scores to electrical output, spectral ratio and coherence values.

### Summary of the Invention

According to one aspect of the present invention, there is provided a method according to claim 1.

According to another aspect of the present invention, there is provided apparatus according to claim 29.

Embodiments of the invention described herein provide a method of diagnosing the presence of a neurological disorder (such as Alzheimer's Disease, depression, or schizophrenia), otherwise assessing the neurological condition of a patient, or characterising the results of a treatment regimen used by a patient. The method includes the collection and analysis of ERP data. The method begins by conducting a plurality of ERP trials on a patient. In an embodiment of the invention, the data from the ERP trials is then characterised to produce a single characterising ERP signal vector for the patient. This reduces the artifacts and noise level in the data. Projections based on the characterising ERP signal vector are then generated. The projections are compared to information derived from the ERP data of patients having known neurological conditions. To perform diagnosis, for example, the projections are compared to standards, such as one or more characterising ERP signal vectors from known healthy patients, and one or more characterising ERP signal vectors from patients known to have the disorder. The probable presence or absence of the neurological disorder is decided by a weighted vote of the projections, where the weighting is a function of how closely each projection compares to the respective standards. Projections can also be used to perform other types of neurological assessment, such as tracking a patient's response to a treatment regimen, assessing the treatability of a patient with respect to a particular regimen, or determining the effects of a particular regimen.

### Brief Description of the Figures

The foregoing and other features and advantages of the embodiments the invention will be apparent from the following, more particular description of a preferred embodiment of the invention, as illustrated in the accompanying drawings.

FIG. 1 is a flowchart illustrating the diagnosis process, according to an embodiment of the invention.

FIG. 2 is a flowchart illustrating the step of characterising ERP data, according to an embodiment of the invention.

FIG. 3 illustrates the process of concatenating waveforms that represent ERP signals collected by different electrodes, according to an embodiment of the invention.

FIG. 4 illustrates the process of sampling a concatenated signal to produce a vector of amplitude values, according to an embodiment of the invention.

FIG. 5 is a flowchart illustrating the step of conducting a weighted vote to facilitate a diagnosis, according to an embodiment of the invention.

FIG. 6 is a flowchart illustrating the process for assessing the treatability of a patient, according to an embodiment of the invention.

FIG. 7 is a flowchart illustrating the process for assessing the response of a patient to a treatment regimen, according to an embodiment of the invention.

FIG. 8 is a flowchart illustrating the process for assessing the nature and extent of side effects resulting from a treatment regimen, according to an embodiment of the invention.

FIG. 9 is a flowchart illustrating the process for characterizing the results of a treatment regimen.

FIG.10 is a flowchart illustrating a generalized method of an embodiment of the invention.

FIG.11 illustrates an exemplary computer system that executes a software embodiment of the invention.

### Detailed Description of the Preferred Embodiments

A preferred embodiment of the present invention is now described with reference to the figures where like reference numbers indicate identical or functionally similar elements. Also in the figures, the left most digit of each reference number corresponds to the figure in which the reference number is first used. While specific configurations and arrangements are discussed, it should be understood that this is done for illustrative purposes only. A person skilled in the relevant art will recognize that other configurations and arrangements can be used without departing from the spirit and scope of the invention. It will be apparent to a person skilled in the relevant art that this invention can also be employed in a variety of other devices and applications.

### I. Overview

The invention provides a method of diagnosing a neurological disorder or otherwise assessing the neurological condition of a patient. The method begins with the collection of ERP signals from the patient under assessment, using multiple trials. In an embodiment of the invention, the ERP signals are combined over all trials performed on the patient and characterized. This forms a single characterizing ERP signal vector of the patient under assessment. A series of projections ofthe vector are then created by eliminating components ofthe vector. An assessment can then be made as to the neurological condition of the patient.

Diagnosis, for example, can be performed by comparing the projections to ERP signals collected from one or more known healthy patients, in an embodiment of the invention. In this embodiment, projections of the vector are also compared to ERP signals collected from one or more patients known to have the neurological disorder. A determination can then be made as to whether the patient under assessment is afflicted with the disorder, based on these comparisons. In other applications of the invention, a patient's projections can be used in tracking the patient's response to a treatment regimen, assessing the treatability of the patient with respect to a particular regimen, or determining the effects experienced by a patient as a result of a particular regimen. The invention can also be used to evaluate a specific treatment regimen, such as a new drug, by assessing the response of a patient to the treatment.

### II. Method of Diagnosis

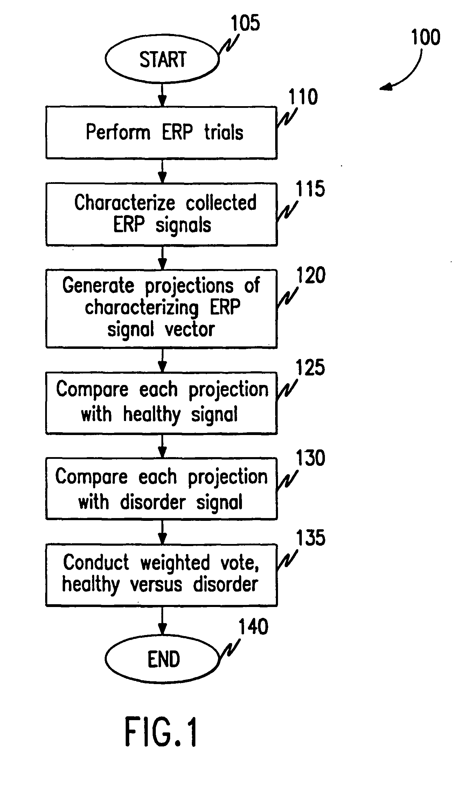

The method of the invention as used in diagnosis, according to one embodiment, is illustrated in flowchart 100 of FIG. 1. The process begins with a step 105. In a step 110, ERPs are elicited from a patient undergoing diagnosis. An ERP represents neural electrical activity that follows a sensory stimulus. Examples of such a stimulus are a flash of light or an auditory tone. A single stimulus and the electrical activity that follows constitute a trial. In an embodiment of the invention, a stimulus is presented every one to two seconds over an interval of approximately two minutes. The electrical activity is measured by a set of approximately 20 electrodes, each of which measures the voltage, or potential, about every ten milliseconds (msec). The result is a set of ERP signals, or waveforms, for each electrode, where each waveform represents a response to an instance of a stimulus. Each waveform is therefore approximately one to two seconds in length.

In a step 115, these ERP signals are combined and characterized to form a single characterizing ERP signal vector for the patient under assessment. Such a vector represents the patent's overall response to sensory stimuli. The characterization is performed in such a manner as to reduce extraneous artifacts and improve the SNR of the ERP data. In an embodiment described below, ERP signals are averaged over multiple trials. Other embodiments can use other forms of characterization, provided that the characterization operation considers all the ERP signals. Moreover, the characterizing ERP signal vector resulting from such a process must reflect the patient's tendencies with respect to the ERPs while minimizing noise and artifacts.

In a step 120, projections of the characterizing ERP signal vector are generated. A projection of a vector is produced when some of the coordinates of the vector are eliminated. In an embodiment of the invention, the coordinates that are eliminated are randomly chosen. In an alternative embodiment, the coordinates to be eliminated are chosen in a deterministic manner. In either case, the result is a group of projections, where each projection is a vector having fewer coordinates than the original characterizing ERP signal vector. If the characterizing ERP signal vector is viewed as a point in an information space, then this step serves to project the characterizing ERP signal vector into information subspaces of lower dimension.

Note that, in an alternative embodiment of the invention, projections are generated based on the ERP signals themselves rather than on a characterization of the ERP signals. In such an embodiment, the characterization step 115 is not necessary. Once projections have been generated in step 120, a determination can be made as to whether the patient is likely to be afflicted with the neurological disorder. In an embodiment ofthe invention, this determination includes steps 125 through 135 of flowchart 100. In step 125, each projection is compared to one or more characterizing ERP signal vector of one or more healthy patients, respectively. In step 130, each projection is compared to one or more characterizing ERP signal vectors from one or more patients known to have the neurological disorder. For each projection, the goal is to determine, first, how closely the projection resembles the characterizing ERP signal vectors of one or more healthy patients, and second, how closely the projection resembles the characterizing ERP signal vector of one or more afflicted patients. In an embodiment of the invention, the comparisons are performed by calculating a statistical measure of resemblance (such as correlation) between each projection and the characterizing ERP signal vectors of the healthy and afflicted patients, respectively.

In step 135, a determination is made as to whether the characterizing ERP signal vector of the patient under assessment bears a closer resemblance to that of a healthy patient or that of a patient having the neurological disorder. The determination is made by a weighted vote of the projections. For each characterizing ERP signal vector of a healthy patient, the resemblance statistics (e.g., correlations) of all the projections with respect to the characterizing ERP signal vector of the healthy patient are summed to create a healthy sum. Likewise, for each characterizing ERP signal vector of an afflicted patient, the resemblance statistics of all the projections with respect to the characterizing ERP signal vector of the afflicted patient are summed to create a disorder sum. All healthy and disorder sums are compared to determine whether the patient under assessment more closely resembles one of the healthy patients or more closely resembles one of the afflicted patients. The result of this comparison can then be used in diagnosis, to determine whether the patient under assessment is likely suffering from the disorder. The process concludes with a step 140.

### A. Characterization of ERP Signals

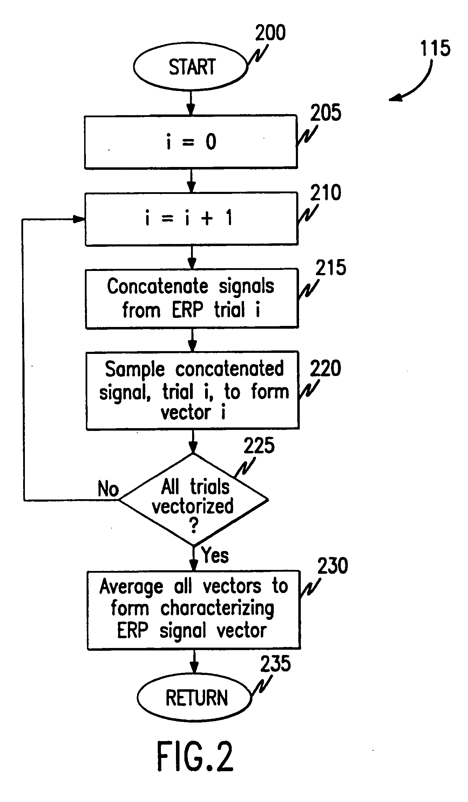

The characterization step 115 is illustrated in greater detail in FIG. 2. In the embodiment shown, the signals captured by each electrode for a given ERP trial are concatenated to form a single waveform. This waveform represents all the ERP data for the trial. The waveform is then sampled to produce a vector. The process is repeated for all trials. The resulting set of vectors includes one vector for each trial. The vectors are then averaged, by coordinate, to produce a characterizing ERP signal vector for the patient.

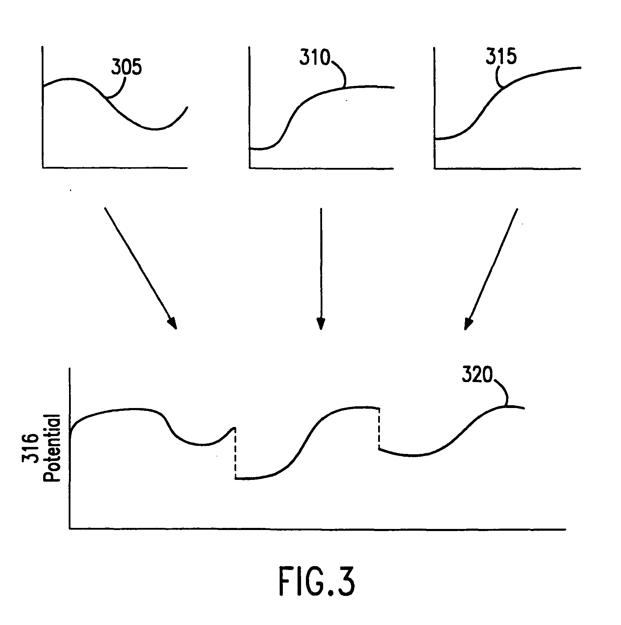

The process begins with a step 200. In steps 205 and 210, an index value i is initialized. Initially, i is set to 1, so that in a step 215, the signals collected by the electrodes during the first trial, trial 1, are concatenated to form a concatenated signal. Step 215 is illustrated graphically in FIG. 3, where signals 305, 310, and 315 are concatenated to form a concatenated signal 320.

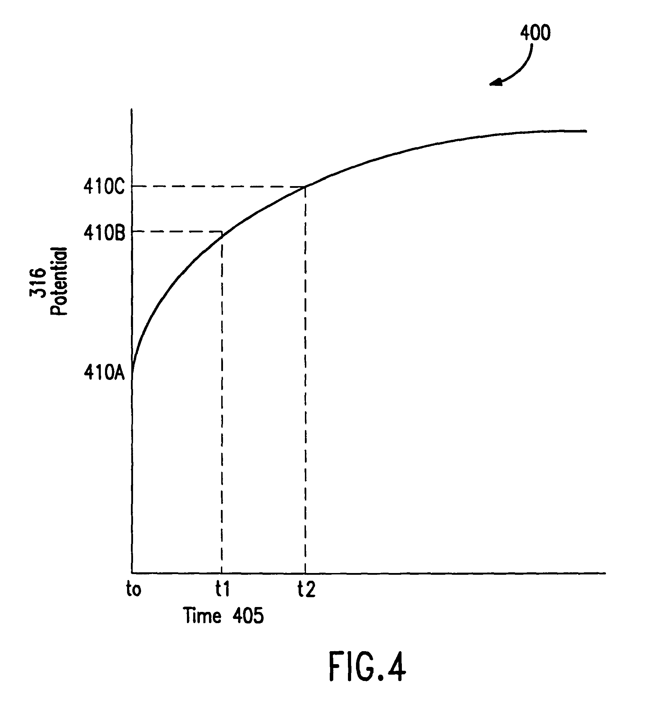

Returning to FIG. 2, in a step 220, the concatenated signal is sampled at a predetermined sampling rate to derive a vector of amplitude values. Sampling step 220 is illustrated graphically in FIG. 4. The amplitude, potential 316, of an example concatenated signal 400 is measured at fixed intervals, at points t₀, t₁, and t₂. The resulting set of amplitudes therefore includes the potentials corresponding to these points. These potentials are identified in FIG. 4 as potentials 410A, 410B, and 410C, respectively. In an embodiment of the invention, sampling is performed at intervals of approximately 10 msec.

Returning to FIG. 2, the set of amplitudes sampled in step 220 are saved in a single vector, vector 1, corresponding to trial 1. In a step 225, a determination is made as to whether the concatenated signal of the final trial has been converted into a vector. If not, the process returns to step 210, where the index i is incremented so that the ERP signals from the next trial can be processed. The ERP signals from the next trial are then concatenated, sampled, and converted into a vector. The process continues until the ERP signals from each trial have been converted into a vector, one vector per trial. The determination as to whether the ERP signals of the final trial have been converted into a vector is made in step 225. In a step 230, the vectors are averaged to form a characterizing ERP signal vector. This is performed on a per component basis. The nth component in the characterizing ERP signal vector is therefore the arithmetic mean of the nth components of all the individual vectors. Because the characterizing ERP signal vector is a function of all the ERP signals collected from the patient being diagnosed, noise and artifacts tend to be minimized. The characterization process 115 concludes with a step 235.

### B. Comparison of a Characterizing ERP Signal Vector to Known Standards

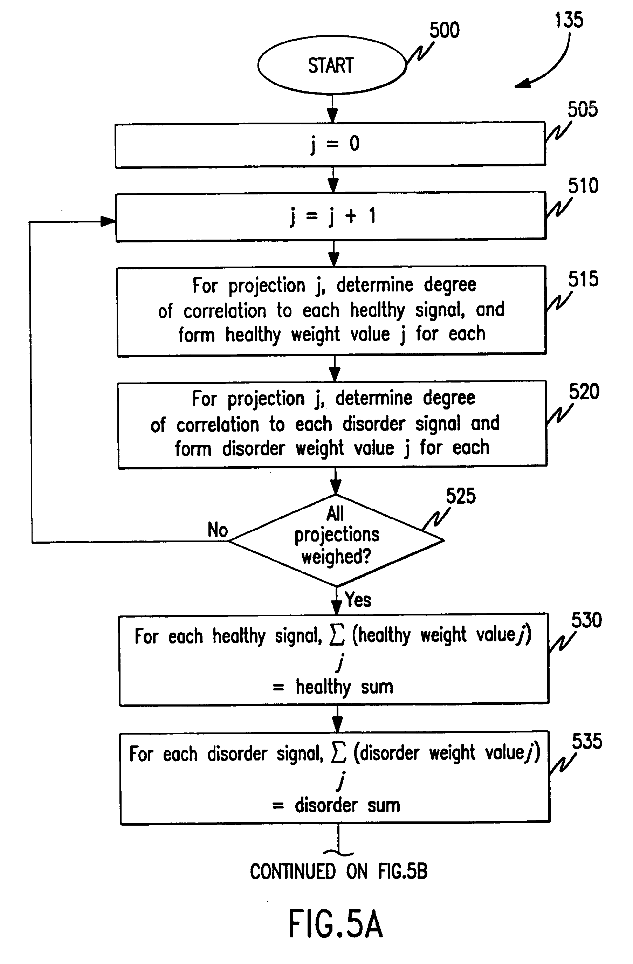

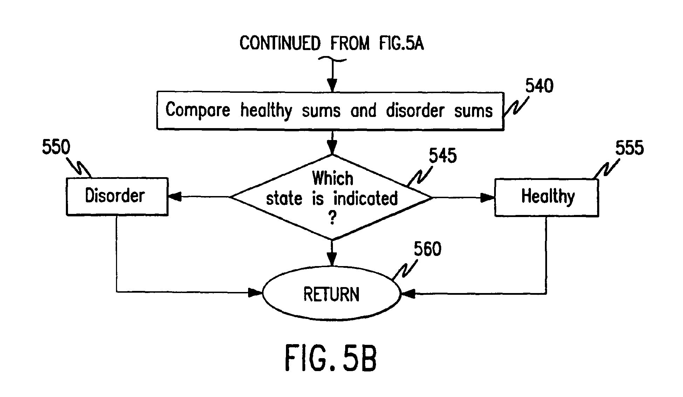

The comparison step 135 is illustrated in greater detail in FIG. 5. In the embodiment illustrated, each projection is compared to one or more characterizing ERP signal vectors of one or more healthy patients, respectively. Each projection is also compared to one or more characterizing ERP signal vectors of one or more afflicted patients, respectively. The corresponding correlations are then derived for each projection. The correlations are used to perform a weighted vote to decide whether or not the patient is more likely to be afflicted with the disorder.

Comparison step 135 begins with a step 500. In steps 505 and 510, an index j is initialized. Index j is used to keep track of the processing of the projections. In a step 515, the first projection, projection 1, is correlated to each of one or more characterizing ERP signal vectors taken from one or more healthy patients, respectively. These characterizing ERP signal vectors are known hereinafter as healthy signals. This results in a correlation statistic, referred to as a healthy weight value 1, for each healthy signal. In a step 520, projection 1 is correlated to one or more characterizing ERP signal vectors of one or more patients, respectively, where these patients are suffering from the neurological disorder. These vectors are known hereinafter as disorder signals. This results in a correlation statistic, referred to as a disorder weight value 1, for each disorder signal is. In a step 525, a determination is made as to whether all the projections have been correlated and their weight values calculated. If not, the process returns to step 510, where the index j is incremented. The correlation process is then performed with respect to the next projection.

If, in step 525, it is determined that all correlations have been performed on all the projections and the weight values determined, then the process continues with a step 530. In step 530, for each healthy signal, the healthy weight values are summed over all the projections. This produces a healthy sum for each healthy signal. In a step 535, for each disorder signal, the disorder weight values are summed over all the projections. This produces a disorder sum for each disorder signal. In a step 540, the healthy sums and the disorder sums are compared. Depending on which state is indicated in a step 545, the patient will be identified as more likely to have the disorder (a step 550) or more likely to be healthy (a step 555). The determination of step 545 is made by analyzing the healthy sums and disorder sums. If the largest sum is a healthy sum, for example, the patient under assessment may be more likely to be healthy. If the highest sum is a disorder sum, on the other hand, the patient under assessment may be more likely to be afflicted with the disorder. The process concludes with a step 560.

### III. Other Applications

The techniques described above can address a range of problems broader than the diagnosis of neurological disorders. Examples of such problems, and the embodiments ofthe invention applicable to these problems, are discussed below.

### A. Assessment of Treatability

An embodiment of the invention can be used to determine whether a patient having a neurological disorder is treatable using a particular treatment regimen. Such a regimen can consist of a particular dosage of a certain drug, such as PROZAC in the case of a patient suffering from depression. In this embodiment ofthe invention, projections of a patient's ERP data are created. The projections are then compared to one or more characterizing ERP signal vectors of one or more patients, respectively, known to be treatable by the particular treatment regimen. If the comparison reveals some degree of similarity between the condition of the patient under assessment and the condition of the patient(s) known to be treatable under the regimen, the treatability of the patient under assessment is suggested.

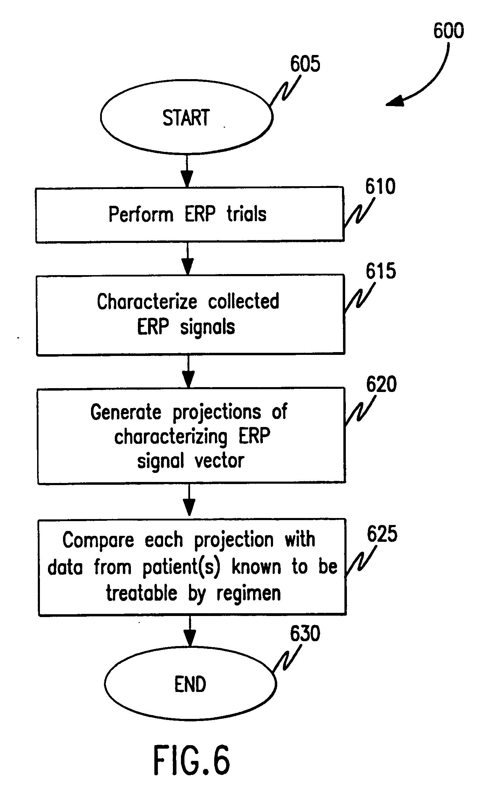

An embodiment of the invention for the assessment of treatability of a patient is illustrated in FIG. 6. The process begins with a step 605. In steps 610 through 620, ERP data is collected, characterized, and processed to produce projections. These steps are analogous to steps 110 through 120 of FIG. 1. A determination is then made as to whether the patient is likely to be treatable given a particular treatment regimen. In the embodiment illustrated, this is done in a step 625 by comparing the projections to information derived from ERP data of one or more patients known to be treatable by the regimen. The projections can, for example, be compared to characterizing ERP signal vectors of the patients known to be treatable by the regimen. For each projection and characterizing ERP signal vector, a degree of correlation between the two is determined and assigned a first weight value. For each patient known to be treatable by the regimen, the first weight values over all projections are summed. For a given patient known to be treatable by the regimen, this yields a sum indicative of the treatability, with respect to the particular regimen, ofthe patient under assessment, relative to the given patient.

Note that this process can also be used to select a particular treatment from among a set of options. The process can be repeated, for example, in relation to a second treatment regimen. This would result in a weight value, known herein as a second weight value, for each projection and each of one or more patients known to be treatable with the second regimen. For a given patient known to be treatable by the second regimen, summing the second weight values over all projections yields a second sum. This latter sum would be indicative of the treatability of the patient under assessment, with respect to the second regimen, relative to the given patient. First sums and second sums can then be compared to evaluate the relative treatability of the patient with respect to the first and second treatment regimens. If, for example, the projections are more similar to patients treatable by one regimen than to patients treatable by the second, then this suggests that the first treatment regimen should be chosen. By analogy, this method can be extended to a plurality of treatment regimens, so that the relative treatablilty ofthe patient can be assessed with respect to this plurality ofregimens.

### B. Assessment of Progress During Treatment

An embodiment of the invention can also be used to assess the progress ofa patient undergoing treatment for a neurological disorder. In this embodiment, projections of a patient's ERP data are created. The projections are compared to one or more characterizing ERP signal vectors of one or more other patients, respectively, where the other patients each have some known neurological condition. If the comparison reveals some degree of similarity between the condition of the patient under assessment and the condition of the other patient(s), then conclusions can be drawn as to the condition of the patient under assessment. If, for example, there is similarity between the condition of the patient under assessment and the condition of a patient who is neurologically healthy, this might suggest improvement in the condition of the patient under assessment. On the other hand, another patient may be afflicted with the same disorder for which the patient under assessment is being treated. Here, similarity between the condition of the patient under assessment and the condition of the afflicted patient might suggest a lack of improvement in the condition of the patient under assessment. Alternatively, another patient may have some other condition, one that represents a potential side effect of the treatment being used on the patient under assessment. Here, similarity between the condition of the patient under assessment and the condition of the patient suffering from the side effect might suggest the presence of the side effect in the patient under assessment.

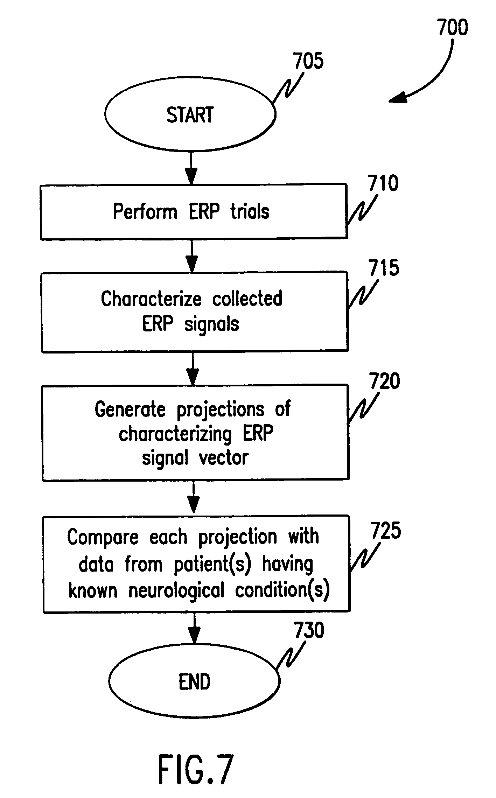

An embodiment of the invention, as applied to assessment of a patient's progress under treatment, is illustrated in FIG. 7. The process begins with a step 705. In steps 710 through 720, ERP data is collected, characterized, and processed to produce projections. These steps are analogous to steps 110 through 120 of FIG. 1. A determination can then be made as to the progress of the patient under assessment. In the illustrated embodiment, this is done in a step 725. Here, each projection is compared with information derived from one or more other patients. In an embodiment ofthe invention, each projection is compared with one or more characterizing ERP signal vectors from one or more other patients, respectively. For each of the characterizing ERP signal vectors of the other patients, the degree of correlation between the characterizing ERP signal vector and each projection is determined. Each correlation is assigned a progress weight value.

Adding together the progress weight values over all projections with respect to the characterizing ERP signal vector of a given patient yields a progress sum that can be indicative of the condition of the patient under assessment, relative to the condition of the given patient. The summation process can be repeated with respect to the other patients as well. This yields a progress sum with respect to each of the other patients. As described above, the progress sums can facilitate inferences as to the progress of the patient under assessment.

### C. Discerning Side Effects of Treatment

An embodiment of the invention can also be used to assess whether a patient undergoing treatment for a neurological disorder is experiencing side effects. This embodiment also allows assessment of the nature and extent of the side effects. In this embodiment, projections of a patient's ERP data are created and compared to characterizing ERP signal vectors of one or more other patients, where these patients each have a known neurological condition, e.g., a condition that represents a possible side effect of the treatment. The degree of similarity (or dissimilarity) between the projections and the characterizing ERP signal vectors of these other patients provides information suggesting the nature and degree of these side effects in the patient under assessment.

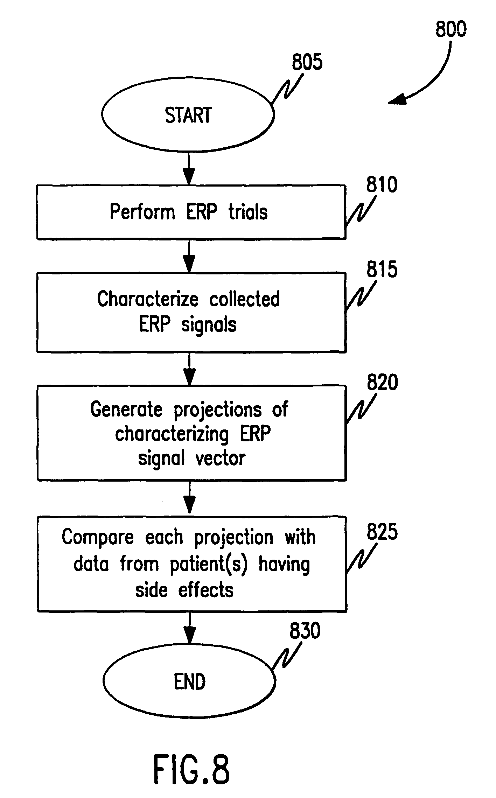

An embodiment of the invention, as applied to assessment of side effects experienced by a patient, is illustrated in FIG. 8. The process begins with a step 805. In steps 810 through 820, ERP data is collected, characterized, and processed to produce projections. These steps are analogous to steps 110 through 120 of FIG. 1. A determination is then made as to whether (and to what extent) the patient is likely suffering from any of a variety of side effects of a particular treatment regimen. In the embodiment illustrated, this is done in a step 825 by comparing the projections to information derived from the ERP data of one or more other patients. At the time the ERP data was collected from these other patients, these patients were known to have certain neurological conditions. For example, they may have been experiencing side effects from the treatment regimen in question. In one embodiment, the projections are compared to characterizing ERP signal vectors of the other patients. For each projection and each characterizing ERP signal vector of another patient, a degree of correlation between the projection and the characterizing ERP signal vector of the other patient is determined and assigned a side effect weight value. For each of the other patients, the side effect weight values over all projections with respect to the characterizing ERP signal vector of the other patient are added together. This yields a set of side effect sums, where each sum is indicative of the similarity of the condition of the patient under assessment to the side effect associated with one of the other patients. This allows evaluation of whether the patient under assessment is likely experiencing a particular side effect or combination of side effects, and the extent to which any given side effect is being experienced.

### D. Evaluation of a Novel Treatment

While the invention can facilitate the assessment of a patient, the invention may also be used in the assessment of treatments themselves. By observing the response of a patient to a novel treatment, observations can be made about the treatment. If, for example, it is suspected that a given drug causes or tends to cause a certain side effect, this suspicion can be confirmed or refuted by using this process to assess a group of test patients. In addition, this process can be used to establish the likelihood that patients will experience a certain side effect, again, by using this invention to assess a group of test patients. This process also permits the discovery of new side effects if, for example, a test patient's projections fail to correlate to ERP data associated with known side effects.

In this embodiment of the invention, projections of a test patient's ERP data are created and compared to characterizing ERP signal vectors of one or more other patients, where these patients each have a known neurological condition. The degree of similarity (or dissimilarity) between the projections and the characterizing ERP signal vectors of these other patients provides information suggesting the effect of the treatment on the test patient.

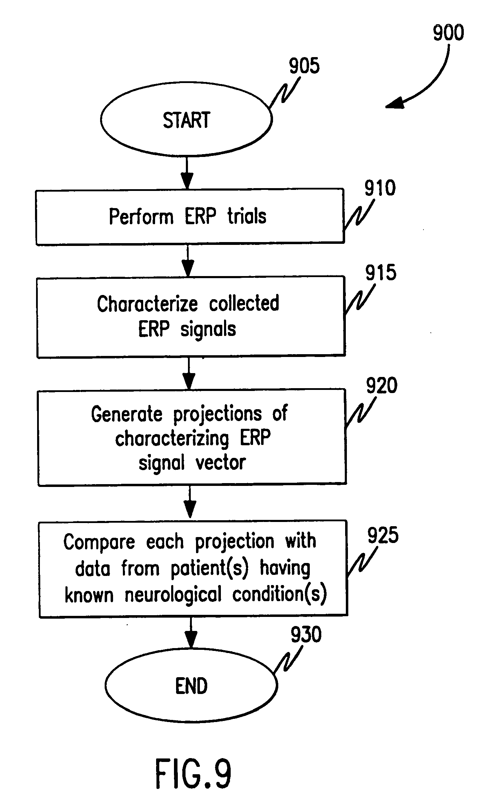

An embodiment of the invention, as applied to assessment of the results of a treatment regimen, is illustrated in FIG. 9. The process begins with a step 905. In steps 910 through 920, ERP data is collected, characterized, and processed to produce projections. These steps are analogous to steps 110 through 120 of FIG. 1. A determination is then made as to the test patient's likely neurological condition as a result of the treatment regimen. In the embodiment illustrated, this is done in a step 925 by comparing the projections to information derived from the ERP data of one or more other patients. At the time the ERP data was collected from these other patients, they were known to have certain neurological conditions. For example, some may have been experiencing some form of neurological disorder, or some may have been neurologically healthy. In one embodiment, the projections are compared to characterizing ERP signal vectors of the other patients. For each projection and each characterizing ERP signal vector of another patient, a degree of correlation between the projection and the characterizing ERP signal vector of the other patient is determined and assigned a comparison weight value.

For each of the other patients, the comparison weight values over all projections with respect to the characterizing ERP signal vector of the other patient are added together. This yields a set of comparison sums, where each comparison sum is indicative of the similarity of the condition of the test patient to the condition of one of the other patients. This allows assessment of the neurological condition of the test patient resulting from the treatment regimen and therefore allows characterization of the treatment's effects.

### E. Generalized Application

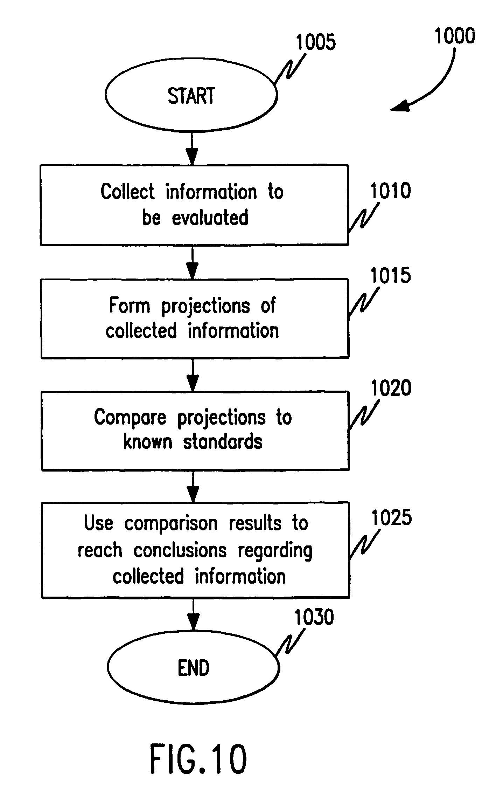

When generalized, the invention allows the processing of a body of information so as to permit conclusions about whether the information can be associated with a particular category or condition. Such an approach can be used, for example, in imaging or signal processing, where an image or signal must be identified or categorized. A general process for this is illustrated in FIG. 10. The process 1000 begins with a step 1005. In a step 1010, the information to be evaluated is collected. In a step 1015, projections of the collected information are created. Projections are formed, in general, by characterizing the collected information and eliminating portions of the characterization. This step projects the characterized information into information subspaces. The projections can then be compared to known standards in a step 1020, where the standards are representative of categories or conditions with which the information may be associated. In a step 1025, the results of the comparisons are used to reach conclusions about whether the collected information can be associated with one of the categories or conditions represented by the standards of step 1020. The process concludes with a step 1030.

### IV. Environment

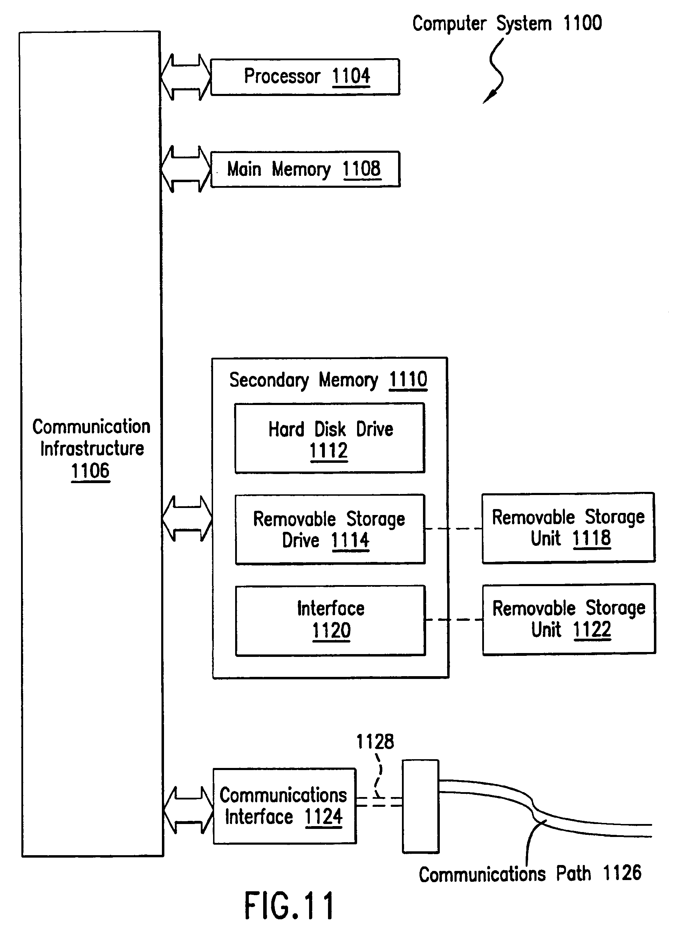

The present invention may be implemented using hardware, software or a combination thereof and may be implemented in a computer system or other processing system. An example of such a computer system 1100 is shown in FIG. 11. The computer system 1000 includes one or more processors, such as processor 1004. The processor 1104 is connected to a communication infrastructure 1106, such as a bus or network). Various software implementations are described in terms of this exemplary computer system. After reading this description, it will become apparent to a person skilled in the relevant art how to implement the invention using other computer systems and/or computer architectures.

Computer system 1100 also includes a main memory 1108, preferably random access memory (RAM), and may also include a secondary memory 1110. The secondary memory 1110 may include, for example, a hard disk drive 1112 and/or a removable storage drive 1114, representing a floppy disk drive, a magnetic tape drive, an optical disk drive, etc. The removable storage drive 1114 reads from and/or writes to a removable storage unit 1118 in a well known manner. Removable storage unit 1118, represents a floppy disk, magnetic tape, optical disk, or other storage medium which is read by and written to by removable storage drive 1114. As will be appreciated, the removable storage unit 1118 includes a computer usable storage medium having stored therein computer software and/or data.

In alternative implementations, secondary memory 1110 may include other means for allowing computer programs or other instructions to be loaded into computer system 1100. Such means may include, for example, a removable storage unit 1122 and an interface 1120. Examples of such means may include a program cartridge and cartridge interface (such as that found in video game devices), a removable memory chip (such as an EPROM, or PROM) and associated socket, and other removable storage units 1122 and interfaces 1120 which allow software and data to be transferred from the removable storage unit 1122 to computer system 1100.

Computer system 1100 may also include a communications interface 1124. Communications interface 1124 allows software and data to be transferred between computer system 1100 and external devices. Examples of communications interface 1124 may include a modem, a network interface (such as an Ethernet card), a communications port, a PCMCIA slot and card, etc. Software and data transferred via communications interface 1124 are in the form of signals 1128 which may be electronic, electromagnetic, optical or other signals capable ofbeing received by communications interface 1124. These signals 1128 are provided to communications interface 1124 via a communications path (i.e., channel) 1126. This channel 1126 carries signals 1128 and may be implemented using wire or cable, fiber optics, a phone line, a cellular phone link, an RF link and other communications channels. In an embodiment ofthe invention, signals 1128 comprise ERP signals collected from a patient and characterizing ERP signal vectors from known healthy and afflicted patients. Alternatively, this information can be provided to computer system 1100 from secondary memory 1110.

In this document, the terms "computer program medium" and "computer usable medium" are used to generally refer to media such as removable storage units 1118 and 1122, a hard disk installed in hard disk drive 1112, and signals 1128. These computer program products are means for providing software to computer system 1100.

Computer programs (also called computer control logic) are stored in main memory 1108 and/or secondary memory 1110. Computer programs may also be received via communications interface 1124. Such computer programs, when executed, enable the computer system 1100 to implement the present invention as discussed herein. In particular, the computer programs, when executed, enable the processor 1104 to implement the present invention. Accordingly, such computer programs represent controllers of the computer system 1100. Where the invention is implemented using software, the software may be stored in a computer program product and loaded into computer system 1100 using removable storage drive 1114, hard drive 1112 or communications interface 1124. In an embodiment of the present invention, steps 115 through 135 of flowchart 100 can be implemented in software and can therefore be made available to processor 1104 through any of these means. Analogously, steps 615 through 625 of flow chart 600, steps 715 through 725 of flow chart 700, steps 815 through 825, and steps 915 through 925 of flow chart 900 can be implemented in software and can therefore be made available to processor 1104 through any of these means.

### V. Conclusion

While various embodiments of the present invention have been described above, it should be understood that they have been presented by way of example, and not limitation. It will be apparent to persons skilled in the relevant art that various changes in detail can be made therein without departing from the scope of the invention. Thus the present invention should not be limited by any of the above-described exemplary embodiments, but should be defined only in accordance with the following claims.

## Claims

1. A method of providing an identification relating to a neurological disorder, comprising the steps of:
(a) receiving (1010) evoked response potential (ERP) data derived from a plurality of ERP trials on a patient under assessment;
(b) generating (1015) a plurality of projections of the ERP data by eliminating components of the vector, **characterised in**;
(c) comparing (1020) each projection with characterizing ERP data projections from other patients, to produce comparison results for each projection; and
(d) determining (1025) an identification relating to the neurological disorder by a weighted vote process that is based on the comparison results.

2. The method of claim 1, wherein the data is derived from ERP trials repeated approximately once every one to two seconds, for approximately two minutes.

3. The method of claim 1, wherein the ERP data comprises ERP signals (305, 310, 315) obtained from the plurality of trials performed on the patient under assessment, the method further comprising, after step (a) and before step (b):
(a1) characterising (115) the ERP signals (400) to produce a characterizing
ERP signal vector (410A, 410B, 410C) for the patient under assessment; and wherein step (b) comprises generating (120) a plurality of projections of the characterizing ERP signal vector of the patient under assessment.

4. The method of claim 3, wherein step (a1) comprises the steps of:
(a1)(i) for each ERP trial, concatenating (215) the signals (305, 310, 315) received by electrodes of an ERP trial apparatus in a predetermined order to form a concatenated signal (320);
(a1)(ii) for each ERP trial, sampling (220) the concatenated signal (320) at a predetermined sampling rate, to form a vector of amplitude values (410A, 410B, 410C) for each ERP trial; and
(a1)(iii) averaging (230) corresponding amplitude values taken from each vector, to form a characterising ERP signal vector of the patient under assessment.

5. The method of claim 4, wherein step (a1)(ii) comprises sampling each concatenated signal (400) approximately once every 10 milliseconds.

6. The method of any one of claims 3 to 5, wherein step (b) comprises randomly deleting components of the characterizing ERP signal vector of the patient under assessment to create the projections.

7. The method of any one of claims 3 to 5, wherein step (b) comprises deleting components of the characterizing ERP signal vector of the patient under assessment according to a deterministic algorithm to create the projections.

8. The method of any preceding claim, wherein the identification relates to the presence of the neurological disorder in the patient under assessment.

9. The method of claim 8, wherein step (c) includes comparing (125) each projection with at least one characterizing ERP signal vector from at least one patient, respectively, known not to be afflicted with the neurological disorder.

10. The method of claim 8 or claim 9, wherein step (c) includes comparing (130) each projection with at least one characterizing ERP signal vector from at least one patient, respectively, known to be afflicted with the neurological disorder.

11. The method of claim 9 or 10, wherein step (d) comprises the steps of:
(d)(i) deriving (515) a healthy weight value for each projection and each characterizing ERP signal vector from a healthy patient, wherein the healthy weight value is determined by a degree of correlation between the projection and the characterizing ERP signal vector from the healthy patient, such that a greater degree of correlation between the projection and the characterizing ERP signal vector from the healthy patient corresponds to a larger healthy weight value;
(d)(ii) deriving (520) a disorder weight value for each projection and each characterizing ERP signal vector from a patient known to have the neurological disorder, wherein the disorder weight value is determined by a degree of correlation between the projection and the characterizing ERP signal vector from the patient known to have the neurological disorder, such that a greater degree of correlation between the projection and the characterizing ERP signal vector from the patient known to have the neurological disorder corresponds to a larger disorder weight value;
(d)(iii) for each characterizing ERP signal vector from a healthy patient, summing (530) the healthy weight values for all projections, to produce a healthy sum corresponding to the associated healthy patient;
(d)(iv) for each characterizing ERP signal vector from a patient known to have a neurological disorder, summing (535) the disorder weight values for all projections, to create a disorder sum corresponding to the associated patient known to have the neurological disorder;
(d)(v) comparing (540) the healthy sums and the disorder sums; and
(d)(vi) deciding (545) on the probable presence of the neurological disorder based on whether any of the disorder sums is greater than any of the healthy sums.

12. The method of any one of claims 1 to 7, wherein the identification relates to the treatability, with respect to a first treatment regimen, of the patient under assessment.

13. The method of claim 12, wherein step (c) includes comparing (625) each projection with at least one characterizing ERP signal vector from at least one patient, respectively, known to be treatable by the first treatment regimen.

14. The method of claim 12, wherein step (d) comprises the steps of:
(d)(i) for each projection and each of at least one characterising ERP signal vector from each of at least one patient, respectively, known to be treatable by the first treatment regimen, deriving a first weight value that is determined by a degree of correlation between the projection and the characterizing ERP signal vector from the patient known to be treatable by the first treatment regimen;
(d)(ii) for each characterizing ERP signal vector from a patient known to be treatable by the first treatment regimen, summing the first weight values for all projections to produce a first sum; and
(d)(iii) deciding on the probable responsiveness of the patient under assessment to the first treatment regimen, based on the first sums.

15. The method of claim 14, wherein said step (d) further comprises the steps of :
(d)(iv) for each projection and each of at least one characterizing ERP signal vector from each of at least one patient, respectively, known to be treatable by a second treatment regimen, deriving a second weight value that is determined by a degree of correlation between the projection and the characterizing ERP signal vector from the patient known to be treatable by the second treatment regimen;
(d)(v) for each characterizing ERP signal vector from a patient known to be treatable by the second treatment regimen, summing the second weight values for all projections to produce a second sum;
(d)(vi) comparing the first sums and the second sums, and
(d)(vii) deciding on the likely responsiveness of the patient under assessment to the first treatment regimen, relative to the likely responsiveness to the second treatment regimen, based on the first and second sums.

16. The method of any one of claims 1 to 7, wherein the identification relates to the current condition of the patient under assessment, as indicated by the projections, after beginning a treatment of the neurological disorder.

17. The method of claim 16, wherein step (c) includes comparing (725) each projection with at least one characterizing ERP signal vector from at least one patient, respectively, having a known neurological condition.

18. The method of claim 17, wherein step (c) includes comparing each projection with at least one characterizing ERP signal vector from at least one patient, respectively, who is neurologically healthy.

19. The method of claim 17, wherein step (c) includes comparing each projection with at least one characterizing ERP signal vector from at least one patient, respectively, who is afflicted with the neurological disorder of the patient being treated.

20. The method of claim 17, wherein step (d) includes:
(d)(i) for each of at least one characterizing ERP signal vector from at least one patient, respectively, and for each projection, deriving a progress weight value, wherein the progress weight value is determined by a degree of correlation between correlation between the projection and the characterizing ERP signal vector from a patient having a known neurological condition;
(d)(ii) for each of at least one characterizing ERP signal vector from at least one patient, respectively, summing the progress weight values for all projections, to produce a progress sum; and
(d)(iii) providing an indication on the probable current condition of the patient being treated, based on the progress sums.

21. The method of any one of claims 1 to 7, wherein the identification relates to the nature and extent of side effects experienced by the patient in response to a treatment regimen for a neurological disorder.

22. The method of claim 21, wherein step (d) comprises comparing (825) each projection with at least one characterizing ERP signal vector from at least one patient, respectively, having at least one known neurological condition, respectively.

23. The method of claim 22, wherein step (d) comprises the steps of:
(d)(i) for each characterizing ERP signal vector from a patient having a known neurological condition and for each projection, deriving a side effect weight value, wherein each side effect weight value is determined by the degree of correlation between the projection and the characterizing ERP signal vector of the patient having a known neurological condition;
(d)(ii) for each characterizing ERP signal vector from a patient having a known neurological condition, summing the side effect weight values for all projections to produce a side effect sum; and
(d)(iii) providing an indication of the probable nature and extent of the side effects, based on the side effect sums.

24. The method of any one of claims 1 to 7, wherein the identification relates to the results of a treatment regime for a neurological disorder.

25. The method of claim 24, wherein step (c) includes comparing (925) each projection with at least one characterizing ERP signal vector from at least one patient, respectively, wherein the at least one patient has the at least one known neurological condition, respectively.

26. The method of claim 25, wherein step (d) comprises the steps of:
(d)(i) for each characterizing ERP signal vector from a patient having a known neurological condition and for each projection, deriving a comparison weight value, wherein each comparison weight value is determined by the degree of correlation between the projection and the characterizing ERP signal vector of the patient having a known neurological condition;
(d)(ii) for each characterizing ERP signal vector from a patient having a known neurological condition, summing the comparison weight values for all projections to produce a comparison sum; and
(d)(iii) providing an indication characterizing the effects of the treatment regime, based on the comparison sums.

27. A computer program including computer program steps arranged to perform the method of any preceding claim when executed by a suitably arranged computer (1100).

28. A computer readable medium (1118, 1122, 1112, 1128) carrying the computer program of claim 27.

29. Apparatus for providing an identification relating to a neurological disorder, comprising:
an evoked response potential trial apparatus (ERP) for performing a plurality of ERP trials on a patient under assessment;
means for generating (1015) a plurality of projections of ERP data derived from the ERP trials by eliminating components of the vector; **characterised in**
means for comparing (1020) each projection with characterizing ERP data projections from other patients, to produce comparison results for each projection; and
means for determining (1025) an identification relating to the neurological disorder by a weighted vote process that is based on the comparison results.

30. The apparatus of claim 29, wherein the performing means is arranged to repeat an ERP trial approximately once every one to two seconds, for approximately two minutes.

31. The apparatus of claim 29, wherein the ERP data comprises ERP signals obtained from the plurality of trials performed on the patient under assessment, the apparatus further comprising:
means for characterising (115) the ERP signals (400) to produce a characterizing ERP signal vector (410A, 410B, 410C) for the patient under assessment; and
wherein the generating means is arranged to generate the plurality of projections from the characterizing ERP signal vector of the patient under assessment.

32. The apparatus of claim 31, wherein the characterising means are arranged to concatenate (215), for each ERP trial, the signals (305, 310, 315) received by electrodes of an ERP trial apparatus in a predetermined order to form a concatenated signal (320), to sample (220), for each ERP trial, the concatenated signal (320) at a predetermined sampling rate, to form a vector of amplitude values (410A, 410B, 410C) for each ERP trial; and to average (230) corresponding amplitude values taken from each vector, to form the characterising ERP signal vector of the patient under assessment.

33. The apparatus of claim 32, wherein the sampling rate is approximately once every 10 milliseconds.

34. The apparatus of any one of claims 31 to 33, wherein the generating means is arranged randomly to delete components of the characterizing ERP signal vector of the patient under assessment to create the projections.

35. The apparatus of any one of claims 31 to 33, wherein the generating means is arranged to delete components of the characterizing ERP signal vector of the patient under assessment according to a deterministic algorithm to create the projections.

36. The apparatus of any one of claims 29-35, wherein the identification relates to the presence of the neurological disorder in the patient under assessment.

37. The apparatus of claim 36, wherein the comparing means is arranged to compare (125) each projection with at least one characterizing ERP signal vector from at least one patient, respectively, known not to be afflicted with the neurological disorder.

38. The apparatus of claim 36 or claim 37, wherein the comparing means is arranged to compare (130) each projection with at least one characterizing ERP signal vector from at least one patient, respectively, known to be afflicted with the neurological disorder.

39. The apparatus of claim 37 or 38, wherein the determining means is arranged to:
derive (515) a healthy weight value for each projection and each characterizing ERP signal vector from a healthy patient, wherein the healthy weight value is determined by a degree of correlation between the projection and the characterizing ERP signal vector from the healthy patient, such that a greater degree of correlation between the projection and the characterizing ERP signal vector from the healthy patient corresponds to a larger healthy weight value;
derive (520) a disorder weight value for each projection and each characterizing ERP signal vector from a patient known to have the neurological disorder, wherein the disorder weight value is determined by a degree of correlation between the projection and the characterizing ERP signal vector from the patient known to have the neurological disorder, such that a greater degree of correlation between the projection and the characterizing ERP signal vector from the patient known to have the neurological disorder corresponds to a larger disorder weight value;
for each characterizing ERP signal vector from a healthy patient, sum (530) the healthy weight values for all projections, to produce a healthy sum corresponding to the associated healthy patient;
for each characterizing ERP signal vector from a patient known to have a neurological disorder, sum (535) the disorder weight values for all projections, to create a disorder sum corresponding to the associated patient known to have the neurological disorder;
compare the healthy sums and the disorder sums; and
decide on the probable presence of the neurological disorder based on whether any of the disorder sums is greater than any of the healthy sums.

40. The apparatus of any one of claims 29 to 35, wherein the identification relates to the treatability, with respect to a first treatment regimen, of the patient under assessment.

41. The apparatus of claim 40, wherein the comparing means is arranged to compare (625) each projection with at least one characterizing ERP signal vector from at least one patient, respectively, known to be treatable by the first treatment regimen.

42. The apparatus of claim 40, wherein the determining means is arranged to:
for each projection and each of at least one characterising ERP signal vector from each of at least one patient, respectively, known to be treatable by the first treatment regimen, derive a first weight value that is determined by a degree of correlation between the projection and the characterizing ERP signal vector from the patient known to be treatable by the first treatment regimen;
for each characterizing ERP signal vector from a patient known to be treatable by the first treatment regimen, sum the first weight values for all projections to produce a first sum; and
decide on the probable responsiveness of the patient under assessment to the first treatment regimen, based on the first sums.

43. The apparatus of claim 42, wherein the determining means is further arranged to:
for each projection and each of at least one characterizing ERP signal vector from each of at least one patient, respectively, known to be treatable by a second treatment regimen, derive a second weight value that is determined by a degree of correlation between the projection and the characterizing ERP signal vector from the patient known to be treatable by the second treatment regimen;
for each characterizing ERP signal vector from a patient known to be treatable by the second treatment regimen, sum the second weight values for all projections to produce a second sum;
compare the first sums and the second sums, and
decide on the likely responsiveness of the patient under assessment to the first treatment regimen, relative to the likely responsiveness to the second treatment regimen, based on the first and second sums.

44. The apparatus of any one of claims 29 to 35, wherein the identification relates to the current condition of the patient under assessment, as indicated by the projections, after beginning a treatment of the neurological disorder.

45. The apparatus of claim 44, wherein the comparing means is arranged to compare (725) each projection with at least one characterizing ERP signal vector from at least one patient, respectively, having a known neurological condition.

46. The apparatus of claim 45, wherein the comparing means is arranged to compare each projection with at least one characterizing ERP signal vector from at least one patient, respectively, who is neurologically healthy.

47. The apparatus of claim 45, wherein the comparing means is arranged to compare each projection with at least one characterizing ERP signal vector from at least one patient, respectively, who is afflicted with the neurological disorder of the patient being treated.

48. The apparatus of claim 45, wherein the determining means is arranged to:
for each of at least one characterizing ERP signal vector from at least one patient, respectively, and for each projection, derive a progress weight value, wherein the progress weight value is determined by a degree of correlation between correlation between the projection and the characterizing ERP signal vector from a patient having a known neurological condition;
for each of at least one characterizing ERP signal vector from at least one patient, respectively, sum the progress weight values for all projections, to produce a progress sum; and
provide an indication on the probable current condition of the patient being treated, based on the progress sums.

49. The apparatus of any one of claims 29 to 35, wherein the identification relates to the nature and extent of side effects experienced by the patient in response to a treatment regimen for a neurological disorder.

50. The apparatus of claim 49, wherein the comparing means is arranged to compare (825) each projection with at least one characterizing ERP signal vector from at least one patient, respectively, having at least one known neurological condition, respectively.

51. The apparatus of claim 50, wherein the determining means is arranged to:
for each characterizing ERP signal vector from a patient having a known neurological condition and for each projection, derive a side effect weight value, wherein each side effect weight value is determined by the degree of correlation between the projection and the characterizing ERP signal vector of the patient having a known neurological condition;
for each characterizing ERP signal vector from a patient having a known neurological condition, sum the side effect weight values for all projections to produce a side effect sum; and
provide an indication of the probable nature and extent of the side effects, based on the side effect sums.

52. The apparatus of any one of claims 29 to 35, wherein the identification relates to the results of a treatment regime for a neurological disorder.

53. The apparatus of claim 52, wherein the comparing means is arranged to compare each projection with at least one characterizing ERP signal vector from at least one patient, respectively, wherein the at least one patient has the at least one known neurological condition, respectively.

54. The apparatus of claim 53, wherein the determining means is arranged to:
for each characterizing ERP signal vector from a patient having a known neurological condition and for each projection, deriving a comparison weight value, wherein each comparison weight value is determined by the degree of correlation between the projection and the characterizing ERP signal vector of the patient having a known neurological condition;
for each characterizing ERP signal vector from a patient having a known neurological condition, sum the comparison weight values for all projections to produce a comparison sum; and
provide an indication characterizing the effects of the treatment regime, based on the comparison sums.

## Patentansprüche

1. Verfahren zur Bereitstellung einer Erkennung bezüglich einer neurologischen Funktionsstörung, umfassend die Schritte:
(a) Empfangen (1010) von Daten von evozierten Potentialen (EP), hergeleitet von mehreren EP-Versuchen an einem Patienten unter Beobachtung;
(b) Erzeugen (1015) mehrerer Prognosen der EP-Daten durch Eliminieren von Komponenten des Vektors, **gekennzeichnet durch**
(c) Vergleichen (1020) jeder Prognose mit charakteristischen Prognosen von EP-Daten von anderen Patienten, um für jede Prognose Vergleichsresultate zu produzieren; und
(d) Bestimmen (1025) einer Erkennung bezüglich der neurologischen Funktionsstörung **durch** einen gewichteten Auswahlprozess, der auf den Vergleichsresultaten basiert.

2. Verfahren nach Anspruch 1, wobei die Daten von EP-Versuchen hergeleitet werden, die ungefähr alle ein oder zwei Sekunden wiederholt werden, für ungefähr zwei Minuten.

3. Verfahren nach Anspruch 1, wobei die EP-Daten EP-Signale (305, 310, 315) umfassen, die im Rahmen von mehreren Versuchen erhalten wurden, die an dem Patienten unter Beobachtung ausgeführt wurden, wobei das Verfahren weiter Folgendes umfasst, nach Schritt (a) und vor Schritt (b):
(a1) Charakterisieren (115) des EP-Signals (400), um einen charakteristischen EP-Signalvektor (410A, 410B, 410C) für den Patienten unter Beobachtung zu erzeugen, und wobei Schritt (b) das Erzeugen (120) mehrerer Prognosen des charakteristischen EP-Signalvektors des Patienten unter Beobachtung umfasst.

4. Verfahren nach Anspruch 3, wobei Schritt (a1) die Schritte umfasst:
(a1)(i) für jeden EP-Versuch Verknüpfen (215) der Signale (305, 310, 315), die durch Elektroden eines EP-Versuchsgeräts in einer vorbestimmten Folge empfangen werden, um ein verknüpftes Signal (320) zu bilden;
(a1)(ii) für jeden EP-Versuch Abfragen (220) des verknüpften Signals (320) in einer vorbestimmten Abtastrate, um von Amplitudenwerten (410A, 410B, 410C) für jeden EP-Versuch einen Vektor zu bilden und
(a1)(iii) Ausmitteln (230) der entsprechenden Amplitudenwerte, die von jedem Vektor genommen werden, um einen charakteristischen EP-Signalvektor des Patienten unter Beobachtung zu bilden.

5. Verfahren nach Anspruch 4, wobei Schritt (a1) (ii) umfasst, jedes verknüpfte Signal (400) ungefähr alle 10 Millisekunden abzufragen.

6. Verfahren nach einem der Ansprüche 3 bis 5, wobei Schritt (b) umfasst, zufällig ausgewählte Komponenten des charakteristischen EP-Signalvektors des Patienten unter Beobachtung zu löschen, um die Prognosen zu erstellen.

7. Verfahren nach einem der Ansprüche 3 bis 5, wobei Schritt (b) umfasst, Komponenten des charakteristischen EP-Signalvektors des Patienten unter Beobachtung entsprechend einem planmäßigen Algorithmus zu löschen, um die Prognosen zu erstellen.

8. Verfahren nach einem der vorhergehenden Ansprüche, wobei die Erkennung auf das Vorhandensein der neurologischen Funktionsstörung im Patienten unter Beobachtung bezogen ist.

9. Verfahren nach Anspruch 8, wobei Schritt (c) das vergleichen (125) jeder Prognose mit zumindest einem charakteristischen EP-Signalvektor von zumindest einem Patienten umfasst, von dem bekannt ist, dass er nicht an der neurologischen Funktionsstörung leidet.

10. Verfahren nach Anspruch 8 oder Anspruch 9, wobei Schritt (c) das Vergleichen (130) jeder Prognose mit zumindest einem charakteristischen EP-Signalvektor von zumindest einem Patienten umfasst, diesbezüglich, von dem bekannt ist, dass er an der neurologischen Funktionsstörung leidet.

11. Verfahren nach Anspruch 9 oder 10, wobei Schritt (d) die Schritte umfasst:
(d)(i) Herleiten (515) eines Gewichtungswertes der Gesundheit für jede Prognose und jeden charakteristischen EP-Signalvektor von einem gesunden Patienten, wobei der Gewichtungswert der Gesundheit durch ein Maß an Übereinstimmung zwischen der Prognose und dem charakteristischen EP-Signalvektor von dem gesunden Patienten bestimmt ist, sodass ein größeres Maß an Übereinstimmung zwischen der Prognose und dem charakteristischen EP-Signalvektor von dem gesunden Patienten einem höheren Gewichtungswert der Gesundheit entspricht,
(d)(ii) Herleiten (520) eines Gewichtungswertes für Funktionsstörung für jede Prognose und jeden charakteristischen EP-Signalvektor von einem Patienten, von dem bekannt ist, dass er unter der neurologischen Funktionsstörung leidet, wobei der Gewichtungswert für Funktionsstörung bestimmt wird durch ein Maß an Übereinstimmung zwischen der Prognose und dem charakteristischen EP-Signalvektor von dem Patienten, von dem bekannt ist, dass er unter der neurologischen Funktionsstörung leidet, derart, dass ein größeres Maß an Übereinstimmung zwischen der Prognose und dem charakteristischen EP-Signalvektor von dem Patienten, von dem bekannt ist, dass er unter der neurologischen Funktionsstörung leidet, einem höheren Gewichtungswert für Funktionsstörung entspricht,
(d)(iii) für jeden charakteristischen EP-Signalvektor von einem gesunden Patienten, Summieren (530) der Gewichtungswerte der Gesundheit für alle Prognosen, um eine Gesundheitssumme zu erzeugen, entsprechend dem zugehörigen gesunden Patienten,
(d)(iv) für jeden charakteristischen EP-Signalvektor von einem Patienten, von dem bekannt ist, dass er unter einer neurologischen Funktionsstörung leidet, Summieren (535) der Gewichtungswerte für Funktionsstörung für alle Prognosen, um eine Funktionsstörungssumme zu erzeugen, entsprechend dem zugehörigen Patienten, von dem bekannt ist, dass er unter der neurologischen Funktionsstörung leidet,
(d)(v) Vergleichen (540) der Gesundheitssummen und der Funktionsstörungssummen und
(d) (vi) Entscheiden (545) über ein mögliches Vorhandensein der neurologischen Funktionsstörung, darauf basierend, ob eine der Funktionsstörungssummen größer ist als eine der Gesundheitssummen.

12. Verfahren nach einem der Ansprüche 1 bis 7, wobei die Erkennung im Zusammenhang mit der Behandelbarkeit steht, mit Bezug auf ein erstes Behandlungsprogramm des Patienten unter Beobachtung.

13. Verfahren nach Anspruch 12, wobei Schritt (c) das Vergleichen (625) jeder Prognose mit zumindest einem charakteristischen EP-Signalvektor von zumindest einem Patienten beinhaltet, von dem bekannt ist, dass er durch das erste Behandlungsprogramm behandelbar ist.

14. Verfahren nach Anspruch 12, wobei Schritt (d) die Schritte umfasst:
(d)(i) für jede Prognose und jedem von zumindest einem charakteristischen EP-Signalvektor von jedem von zumindest einem Patienten, von dem bekannt ist, dass er durch das erste Behandlungsprogramm behandelbar ist, Herleiten eines ersten Gewichtungswertes, der bestimmt wird durch ein Maß an Übereinstimmung zwischen der Prognose und dem charakteristischen EP-Signalvektor von dem Patienten, von dem bekannt ist, dass er durch das erste Behandlungsprogramm behandelbar ist,
(d)(ii) für jeden charakteristischen EP-Signalvektor von einem Patienten, von dem bekannt ist, dass er durch das erste Behandlungsprogramm behandelbar ist, Summieren der ersten Gewichtungswerte für alle Prognosen, um eine erste Summe zu erzeugen und
(d)(iii) Entscheiden über das mögliche Ansprechen des Patienten unter Beobachtung auf das erste Behandlungsprogramm, basierend auf den ersten Summen.

15. Verfahren nach Anspruch 14, wobei der Schritt (d) weiterhin die Schritte umfasst:
(d)(iv) für jede Prognose und jeden von zumindest einem charakteristischen EP-Signalvektor von jedem von zumindest einem Patienten, von dem bekannt ist, dass er durch ein zweites Behandlungsprogramm behandelbar ist, Herleiten eines zweiten Gewichtungswertes, der bestimmt wird durch ein Maß an Übereinstimmung zwischen der Prognose und dem charakteristischen EP-Signalvektor von einem Patienten, von dem bekannt ist, dass er durch ein zweites Behandlungsprogramm behandelbar ist,
(d)(v) für jeden charakteristischen EP-Signalvektor von einem Patienten, von dem bekannt ist, dass er durch ein zweites Behandlungsprogramm behandelbar ist, Summieren der zweiten Gewichtungswerte für alle Prognosen, um eine zweite Summe zu erzeugen,
(d)(vi) Vergleichen der ersten Summen und der zweiten Summen und
(d)(vii) Entscheiden über das wahrscheinliche Ansprechen des Patienten unter Beobachtung auf das erste Behandlungsprogramm, im Vergleich zu dem wahrscheinlichen Ansprechen auf das zweite Behandlungsprogramm, basierend auf den ersten und zweiten Summen.

16. Verfahren nach einem der Ansprüche 1 bis 7, wobei die Erkennung auf den momentanen Zustand des Patienten unter Beobachtung bezogen ist, wie durch die Prognosen angezeigt, nach dem Beginn einer Behandlung der neurologischen Funktionsstörung.

17. Verfahren nach Anspruch 16, wobei Schritt (c) das Vergleichen (725) jeder Prognose mit zumindest einem charakteristischen EP-Signalvektor von zumindest einem Patienten beinhaltet, der einen bekannten neurologischen Zustand hat.

18. Verfahren nach Anspruch 17, wobei Schritt (c) das Vergleichen jeder Prognose mit zumindest einem charakteristischen EP-Signalvektor von zumindest einem Patienten beinhaltet, der neurologisch gesund ist.

19. Verfahren nach Anspruch 17, wobei Schritt (c) das Vergleichen jeder Prognose mit zumindest einem charakteristischen EP-Signalvektor von zumindest einem Patienten beinhaltet, der unter der neurologischen Funktionsstörung des Patienten, der behandelt wird, leidet.

20. Verfahren nach Anspruch 17, wobei Schritt (d) beinhaltet:
(d)(i) für zumindest einen charakteristischen EP-Signalvektor von zumindest einem Patienten und für jede Prognose, Herleiten eines Gewichtungswertes des Fortschritts, wobei der Gewichtungswert des Fortschritts bestimmt wird durch ein Maß an Übereinstimmung zwischen der Prognose und dem charakteristischen EP-Signalvektor von einem Patienten, der einen bekannten neurologischen Zustand hat,
(d) (ii) für zumindest einen charakteristischen EP-Signalvektor von zumindest einem Patienten, Summieren der Gewichtungswerte des Fortschritts für alle Prognosen, um eine Fortschrittssumme zu erzeugen und
(d)(iii) Bereitstellen einer Erkennung des möglichen momentanen Zustands des Patienten, der behandelt wird, basierend auf den Fortschrittssummen.

21. Verfahren nach einem der Ansprüche 1 bis 7, wobei die Erkennung auf die Art und das Ausmaß der Nebenwirkungen bezogen ist, die bei dem Patienten als Reaktion auf ein Behandlungsprogramm für eine neurologische Funktionsstörung auftritt.

22. Verfahren nach Anspruch 21, wobei Schritt (d) das Vergleichen (825) jeder Prognose mit zumindest einem charakteristischen EP-Signalvektor von zumindest einem Patienten umfasst, der zumindest einen bekannten neurologischen Zustand hat.

23. Verfahren nach Anspruch 22, wobei Schritt (d) die Schritte umfasst:
(d)(i) für jeden charakteristischen EP-Signalvektor von einem Patienten, der einen bekannten neurologischen Zustand hat und für jede Prognose, Herleiten eines Gewichtungswertes für Nebenwirkungen, wobei jeder Gewichtungswert für Nebenwirkungen durch ein Maß an Übereinstimmung zwischen der Prognose und dem charakteristischen EP-Signalvektor des Patienten bestimmt wird, der einen bekannten neurologischen Zustand hat,
(d)(ii) für jeden charakteristischen EP-Signalvektor von einem Patienten, der einen bekannten neurologischen Zustand hat, Summieren des Gewichtungswertes für Nebenwirkungen für alle Prognosen, um eine Nebenwirkungssumme zu erzeugen; und
(d)(iii) Bereitstellen einer Indikation der möglichen Art und des Ausmaßes der Nebenwirkungen, basierend auf der Nebenwirkungssumme.

24. Verfahren nach einem der Ansprüche 1 bis 7, wobei die Erkennung auf die Ergebnisse eines Behandlungsprogramms für eine neurologische Funktionsstörung bezogen ist.

25. Verfahren nach Anspruch 24, wobei Schritt (c) das Vergleichen (925) jeder Prognose mit zumindest einem charakteristischen EP-Signalvektor von zumindest einem Patienten beinhaltet, wobei der zumindest eine Patient den zumindest einen bekannten neurologischen Zustand hat.

26. Verfahren nach Anspruch 25, wobei Schritt (d) die Schritte umfasst:
(d)(i) für jeden charakteristischen EP-Signalvektor von einem Patienten, der einen bekannten neurologischen Zustand hat und für jede Prognose, Herleiten eines Gewichtungswertes für einen Vergleich, wobei jeder Gewichtungswert für einen Vergleich durch ein Maß an Übereinstimmung zwischen der Prognose und dem charakteristischen EP-Signalvektor des Patienten bestimmt ist, der einen bekannten neurologischen Zustand hat,
(d)(ii) für jeden charakteristischen EP-Signalvektor von einem Patienten, der einen bekannten neurologischen Zustand hat, Summieren der Gewichtungswerte für einen Vergleich für alle Prognosen, um eine Vergleichssumme zu erzeugen; und
(d)(iii) Bereitstellen einer Indikation, die die Wirkungen des Behandlungsprogramms basierend auf den Vergleichssummen charakterisiert.

27. Computerprogramm, das Schritte eines Computerprogramms beinhaltet, die vorgesehen sind, um ein Verfahren nach einem der vorhergehenden Ansprüche auszuführen, wenn es durch einen passend eingerichteten Computer (1100) ausgeführt wird.

28. Computerlesbares Medium (1118, 1122, 1112, 1128), das das Computerprogramm nach Anspruch 27 mitführt.

29. Vorrichtung zur Bereitstellung einer Erkennung bezüglich einer neurologischen Funktionsstörung, umfassend:
eine Versuchsvorrichtung für evozierte Potentiale (EP) zur Durchführung mehrerer EP-Versuche an einem Patienten unter Beobachtung,
Mittel zum Erzeugen (1050) mehrerer Prognosen von EP-Daten, hergeleitet von EP-Versuchen durch das Eliminieren von Komponenten des Vektors, **gekennzeichnet durch** Mittel zum Vergleichen (1020) jeder Prognose mit charakteristischen EP-Datenprognosen von anderen Patienten, um für jede Prognose Vergleichsresultate zu erzeugen; und
Mittel zum Bestimmen (1025) einer Erkennung bezüglich der neurologische Funktionsstörung **durch** einen gewichteten Auswahlprozess, der auf den Vergleichsresultaten basiert.

30. Vorrichtung nach Anspruch 29, wobei die ausführenden Mittel angeordnet sind, um einen EP-Versuch ungefähr einmal alle ein oder zwei Sekunden zu wiederholen, für ein oder zwei Minuten.

31. Vorrichtung nach Anspruch 29, wobei die EP-Daten EP-Signale umfassen, die im Rahmen von mehreren Versuchen erhalten wurden, die am Patienten unter Beobachtung ausgeführt wurden, wobei die Vorrichtung des Weiteren Folgendes umfasst:
Mittel zum Charakterisieren (115) der EP-Signale (400), um einen charakteristischen EP-Signalvektor (410A, 410B, 410C) für den Patienten unter Beobachtung zu erzeugen, und
wobei die Mittel des Erzeugens angeordnet sind, um mehrere Prognosen vom charakteristischen EP-Signalvektor des Patienten unter Beobachtung zu erzeugen.

32. Vorrichtung nach Anspruch 31, wobei die Mittel zum Charakterisieren für jeden EP-Versuch zum Verknüpfen (215) der Signale (305, 310, 315) angeordnet sind, die über Elektroden eines EP-Versuchsgeräts in einer vorbestimmten Folge empfangen werden, um ein verknüpftes Signal (320) zu bilden, zum Abfragen (220), für jeden EP-Versuch, der verknüpften Signale (320) in einer vorbestimmten Abtastrate, um einen Vektor von Amplitudenwerten (410A, 410B, 410C) für jeden EP-Versuch zu bilden und zum Ausmitteln (230) entsprechender Amplitudenwerte, die von jedem Vektor entnommen werden, um den charakteristischen EP-Signalvektor für den Patienten unter Beobachtung zu bilden.

33. Vorrichtung nach Anspruch 32, wobei die Abtastung ungefähr einmal alle 10 Millisekunden erfolgt.

34. Vorrichtung nach einem der Ansprüche 31 bis 33, wobei die Mittel der Erzeugung angeordnet sind, um zufallsartig Komponenten des charakteristischen EP-Signalvektors des Patienten unter Beobachtung zu löschen, um die Prognosen zu erzeugen.

35. Vorrichtung nach einem der Ansprüche 31 bis 33, wobei die Mittel der Erzeugung angeordnet sind, um die Komponenten des charakteristischen EP-Signalvektors des Patienten unter Beobachtung gemäß einem planmäßigen Algorithmus zu löschen, um die Prognosen zu erzeugen.

36. Vorrichtung nach einem der Ansprüche 29 bis 35, wobei die Erkennung auf das Vorhandensein der neurologischen Funktionsstörung im Patienten unter Beobachtung bezogen ist.

37. Vorrichtung nach Anspruch 36, wobei die Mittel des Vergleichens zum Vergleichen (125) jeder Prognose mit zumindest einem charakteristischen EP-Signalvektor von zumindest einem Patienten angeordnet sind, von dem bekannt ist, dass er nicht unter der neurologischen Funktionsstörung leidet.

38. Vorrichtung nach Anspruch 36 oder Anspruch 37, wobei die Mittel des Vergleichens zum Vergleichen (130) jeder Prognose mit zumindest einem charakteristischen EP-Signalvektor von zumindest einem Patienten angeordnet sind, von dem bekannt ist, dass er unter der neurologischen Funktionsstörung leidet.

39. Vorrichtung nach Anspruch 37 oder 38, wobei die Mittel zur Bestimmung angeordnet sind zum:
Herleiten (515) eines Gewichtungswertes für Gesundheit für jede Prognose und jeden charakteristischen EP-Signalvektor von einem gesunden Patienten, wobei der Gewichtungswert für Gesundheit bestimmt wird durch ein Maß an Übereinstimmung zwischen der Prognose und dem charakteristischen EP-Signalvektor des gesunden Patienten, sodass ein größeres Maß an Übereinstimmung zwischen der Prognose und dem charakteristischen EP-Signalvektor von dem gesunden Patienten einem höheren Gewichtungswert für Gesundheit entspricht,
Herleiten (520) eines Gewichtungswertes für Funktionsstörung für jede Prognose und jedem charakteristischen EP-Signalvektor von einem Patienten, von dem bekannt ist, dass er unter der neurologischen Funktionsstörungen leidet, wobei der Gewichtungswert der Funktionsstörung bestimmt wird durch ein Maß an Übereinstimmung zwischen der Prognose und dem charakteristischen EP-Signalvektor von dem Patienten, von dem bekannt ist, dass er unter der neurologischen Funktionsstörung leidet, sodass ein größeres Maß an Übereinstimmung zwischen der Prognose und dem charakteristischen EP-Signalvektor von dem Patienten, von dem bekannt ist, dass er unter der neurologischen Funktionsstörung leidet, einem höheren Gewichtungswert für Funktionsstörung entspricht,
für jeden charakteristischen EP-Signalvektor von einem gesunden Patienten, Summieren (530) der Gewichtungswerte für Gesundheit für alle Prognosen, um eine Gesundheitssumme zu erzeugen, die dem zugehörigen gesunden Patienten entspricht,
für jeden charakteristischen EP-Signalvektor von einem Patienten, von dem bekannt ist, dass er unter einer neurologische Funktionsstörung leidet, Summieren (535) der Gewichtungswerte für Funktionsstörung für alle Prognosen, um eine Funktionsstörungssumme zu erzeugen, die dem zugehörigen Patienten entspricht, von dem bekannt ist, dass er unter der neurologischen Funktionsstörung leidet,
Vergleichen der Gesundheitssummen und der Funktionsstörungssummen und
Entscheiden über das mögliche Vorhandensein der neurologischen Funktionsstörung, darauf basierend, ob eine der Funktionsstörungssummen größer ist als eine der Gesundheitssummen.

40. Vorrichtung nach einem der Ansprüche 29 bis 35, wobei die Erkennung auf die Behandelbarkeit hinsichtlich eines ersten Behandlungsprogramms des Patienten unter Beobachtung bezogen ist.

41. Vorrichtung nach Anspruch 40, wobei die Mittel des Vergleichens zum Vergleichen (625) jeder Prognose mit zumindest einem charakteristischen EP-Signalvektor von zumindest einem Patienten angeordnet sind, von dem bekannt ist, dass er durch das erste Behandlungsprogramm behandelbar ist.

42. Vorrichtung nach Anspruch 40, wobei die Mittel der Bestimmung wie folgt angeordnet sind:
Für jede Prognose und jedem von zumindest einem charakteristischen EP-Signalvektor von jedem von zumindest einem Patienten, von dem bekannt ist, dass er durch ein erstes Behandlungsprogramm behandelbar ist, Herleiten eines ersten Gewichtungswertes, der bestimmt wird durch ein Maß an Übereinstimmung zwischen der Prognose und dem charakteristischen EP-Signalvektor von dem Patienten, von dem bekannt ist, dass er behandelbar ist durch ein erstes Behandlungsprogramm,
für jeden charakteristischen EP-Signalvektor von einem Patienten, von dem bekannt ist, dass er durch ein erstes Behandlungsprogramm behandelbar ist, Summieren der ersten Gewichtungswerte für alle Prognosen, um eine erste Summe zu erzeugen und
Entscheiden über das mögliche Ansprechen des Patienten unter Beobachtung auf das erste Behandlungsprogramm, basierend auf den ersten Summen.

43. Vorrichtung nach Anspruch 42, wobei die Mittel der Bestimmung des Weiteren für Folgendes angeordnet sind:
für jede Prognose und jedem von zumindest einem charakteristischen EP-Signalvektor von jedem von zumindest einem Patienten, von dem bekannt ist, dass er durch ein zweites Behandlungsprogramm behandelbar ist, Herleiten eines zweiten Gewichtungswertes, der bestimmt wird durch ein Maß an Übereinstimmung zwischen der Prognose und dem charakteristischen EP-Signalvektor von dem Patienten, von dem bekannt ist, dass er durch ein zweites Behandlungsprogramm behandelbar ist,
für jeden charakteristischen EP-Signalvektor von einem Patienten, von dem bekannt ist, dass er durch ein zweites Behandlungsprogramm behandelbar ist, Summieren der zweiten Gewichtungswerte für alle Prognosen, um eine zweite Summe zu erzeugen,
Vergleichen der ersten Summen und der zweiten Summen und
Entscheiden über das wahrscheinliche Ansprechen des Patienten unter Beobachtung auf das erste Behandlungsprogramm im Vergleich zu dem wahrscheinlichen Ansprechen auf das zweite Behandlungsprogramm, basierend auf den ersten und zweiten Summen.

44. Vorrichtung nach einem der Ansprüche 29 bis 35, wobei die Erkennung auf den momentanen Zustand des Patienten unter Beobachtung bezogen ist, wie durch die Prognosen angezeigt, nach dem Beginn einer Behandlung der neurologische Funktionsstörung.

45. Vorrichtung nach Anspruch 44, wobei die Mittel des Vergleichens zum Vergleichen (725) jeder Prognose mit zumindest einem charakteristischen EP-Signalvektor von zumindest einem Patienten angeordnet sind, der einen bekannten neurologischen Zustand hat.

46. Vorrichtung nach Anspruch 45, wobei die Mittel des Vergleichens zum Vergleichen von jeder Prognose mit zumindest einem charakteristischen EP-Signalvektor von zumindest einem Patienten angeordnet sind, der neurologisch gesund ist.

47. Vorrichtung nach Anspruch 45, wobei die Mittel des Vergleichens zum Vergleichen jeder Prognose mit zumindest einem charakteristischen EP-Signalvektor von zumindest einem Patienten angeordnet sind, der unter der neurologischen Funktionsstörung des Patienten, der behandelt wird, leidet.

48. Vorrichtung nach Anspruch 45, wobei die Mittel der Bestimmung für Folgendes angeordnet sind:
für jeden von zumindest einem charakteristischen EP-Signalvektor von zumindest einem Patienten und für jede Prognose, Herleiten eines Gewichtungswertes für Fortschritt, wobei der Gewichtungswert bestimmt wird durch ein Maß an Übereinstimmung zwischen der Prognose und dem charakteristischen EP-Signalvektor von einem Patienten, der einen bekannten neurologischen Zustand hat,
für jeden von zumindest einem charakteristischen EP-Signalvektor von zumindest einem Patienten, Summieren der Gewichtungswerte für Fortschritt für alle Prognosen, um eine Fortschrittssumme zu erzeugen und
Bereitstellen einer Indikation des möglichen momentanen Zustands des Patienten, der behandelt wird, basierend auf den Fortschrittssummen.

49. Vorrichtung nach einem der Ansprüche 29 bis 35, wobei die Erkennung auf die Art und das Ausmaß der Nebenwirkungen bezogen ist, die beim dem Patienten als Reaktion auf das Behandlungsprogramm für eine neurologische Funktionsstörung auftritt.

50. Vorrichtung nach Anspruch 49, wobei die Mittel des Vergleichens zum Vergleichen (825) jeder Prognose mit zumindest einem charakteristischen EP-Signalvektor von zumindest einem Patienten angeordnet sind, der zumindest einen bekannten neurologischen Zustand diesbezüglich hat.

51. Vorrichtung nach Anspruch 50, wobei die Mittel der Bestimmung für Folgendes angeordnet sind:
Für jeden charakteristischen EP-Signalvektor von einem Patienten, der einen bekannten neurologischen Zustand hat und für jede Prognose, Herleiten eines Gewichtungswertes für Nebenwirkungen, wobei jeder Gewichtungswert durch ein Maß an Übereinstimung zwischen der Prognose und dem charakteristischen EP-Signalvektor des Patienten bestimmt ist, der einen bekannten neurologischen Zustand hat,
für jeden charakteristischen EP-Signalvektor eines Patienten, der einen bekannten neurologischen Zustand hat, Summieren der Gewichtungswerte für Nebenwirkungen für alle Prognosen, um eine Nebenwirkungssumme zu erzeugen und
Bereitstellen einer Indikation der möglichen Art und des Ausmaßes der Nebenwirkungen, basierend auf den Nebenwirkungssummen.

52. Vorrichtung nach einem der Ansprüche 29 bis 35, wobei die Erkennung auf die Resultate eines Behandlungsprogramms für eine neurologische Funktionsstörung bezogen ist.

53. Vorrichtung nach Anspruch 52, wobei die Mittel des Vergleichens zum Vergleichen jeder Prognose mit zumindest einem charakteristischen EP-Signalvektor von zumindest einem Patienten angeordnet sind, wobei der zumindest eine Patient den zumindest einen bekannten neurologischen Zustand hat.

54. Vorrichtung nach Anspruch 53, wobei die Mittel der Bestimmung für Folgendes angeordnet sind:
für jeden charakteristischen EP-Signalvektor von einem Patienten, der einen bekannten neurologischen Zustand hat und für jede Prognose, Herleiten eines Gewichtungswertes für Vergleiche, wobei jeder Gewichtungswert für Vergleiche bestimmt wird durch ein Maß an Übereinstimmung zwischen der Prognose und dem charakteristischen EP-Signalvektor des Patienten, der einen bekannten neurologischen Zustand hat,
für jeden charakteristischen EP-Signalvektor eines Patienten, der einen bekannten neurologischen Zustand hat, Summieren der Gewichtungswerte für alle Prognosen, um eine Vergleichssumme zu erzeugen und
Bereitstellung einer Anzeige, die die Wirkungen des Behandlungsprogramms charakterisiert, basierend auf den Vergleichssummen.

## Revendications

1. Procédé pour fournir une identification se rapport à un trouble neurologique, comportant les étapes consistant à :
(a) recevoir (1010) des données de potentiel de réponse évoqué (ERP) dérivées de plusieurs essais ERP sur un patient sous évaluation;
(b) générer (1015) plusieurs projections des données ERP en éliminant des composantes du vecteur, **caractérisé par**
(c) la comparaison (1020) de chaque projection à des projections de données ERP de caractérisation d'autres patients, pour produire des résultats de comparaison pour chaque projection ; et
(d) la détermination (1025) d'une identification relative au trouble neurologique par un traitement de vote pondéré qui est basé sur les résultats de comparaison.

2. Procédé selon la revendication 1, dans lequel les données sont dérivées à partir d'essais ERP répétés approximativement toutes les une à deux secondes, pendant approximativement deux minutes.

3. Procédé selon la revendication 1, dans lequel les données ERP comportent des signaux ERP (305, 310, 315) obtenus dans les essais effectués sur le patient sous évaluation, le procédé comportant de plus, après l'étape (a) et avant l'étape (b) l'étape consistant à :
(a1) caractériser (115) les signaux ERP (400) pour produire un vecteur de signal ERP de caractérisation (410A, 410B, 410C) pour le patient sous évaluation ;
et dans lequel l'étape (b) comporte la génération (120) de plusieurs projections du vecteur de signal ERP de caractérisation du patient sous évaluation.

4. Procédé selon la revendication 3, dans lequel l'étape (a1) comporte les étapes consistant à :
(a1) (i) pour chaque essai ERP, concaténer (215) les signaux (305, 310, 315) reçus par des électrodes d'un appareil d'essai ERP dans un ordre prédéterminé pour former un signal concaténé (320);
(a1)(ii) pour chaque essai ERP, échantillonner (220) le signal concaténé (320) à une fréquence d'échantillonnage prédéterminée, pour former un vecteur de valeurs d'amplitude (410A, 410B, 410C) pour chaque essai ERP ; et
(a1)(iii) établir la moyenne (230) des valeurs d'amplitude correspondantes prises dans chaque vecteur, pour former un vecteur de signal ERP de caractérisation du patient sous évaluation.

5. Procédé selon la revendication 4, dans lequel l'étape (a1)(ii) comporte l'échantillonnage de chaque signal concaténé (400) approximativement d'une fois toutes les 10 millisecondes.

6. Procédé selon l'une quelconque des revendications 3 à 5, dans lequel l'étape (b) comporte la suppression de manière aléatoire de composantes du vecteur de signal ERP de caractérisation du patient sous évaluation pour créer les projections.

7. Procédé selon l'une quelconque des revendications 3 à 5, dans lequel l'étape (b) comporte la suppression de composantes du vecteur de signal ERP de caractérisation du patient sous évaluation conformément à un algorithme déterministe pour créer les projections.

8. Procédé selon l'une quelconque des revendications précédentes, dans lequel l'identification concerne la présence du trouble neurologique chez le patient sous évaluation.

9. Procédé selon la revendication 8, dans lequel l'étape (c) inclut la comparaison (125) de chaque projection à au moins un vecteur de signal ERP de caractérisation d'au moins un patient, respectivement, connu comme n'étant pas affecté par le trouble neurologique.

10. Procédé selon la revendication 8 ou la revendication 9, dans lequel l'étape (c) inclut la comparaison (130) de chaque projection à au moins un vecteur de signal ERP de caractérisation d'au moins un patient, respectivement, connu comme étant affecté par le trouble neurologique.

11. Procédé selon la revendication 9 ou 10, dans lequel l'étape (d) comporte les étapes consistant à :
(d)(i) dériver (515) une valeur de pondération de bonne santé pour chaque projection et chaque vecteur de signal ERP de caractérisation d'un patient en bonne santé, où la valeur de pondération de bonne santé est déterminée par un degré de corrélation entre la projection et le vecteur de signal ERP de caractérisation du patient en bonne santé, de sorte qu'un plus grand degré de corrélation entre la projection et le vecteur de signal ERP de caractérisation du patient en bonne santé correspond à une plus grande valeur de pondération de bonne santé ;
(d)(ii) dériver (520) une valeur de pondération de trouble pour chaque projection et chaque vecteur de signal ERP de caractérisation d'un patient connu comme ayant le trouble neurologique, où la valeur de pondération de trouble est déterminée par un degré de corrélation entre la projection et le vecteur de signal ERP de caractérisation du patient connu comme ayant le trouble neurologique, de sorte qu'un plus grand degré de corrélation entre la projection et le vecteur de signal ERP de caractérisation du patient connu comme ayant le trouble neurologique correspond à une plus grande valeur de pondération de trouble ;
(d)(iii) pour chaque vecteur de signal ERP de caractérisation d'un patient en bonne santé, additionner (530) les valeurs de pondération de bonne santé pour toutes les projections, pour produire une somme de bonne santé correspondant au patient en bonne santé associé;
(d)(iv) pour chaque vecteur de signal ERP de caractérisation d'un patient connu comme ayant un trouble neurologique, additionner (535) les valeurs de pondération de trouble pour toutes les projections, pour créer une somme de trouble correspondant au patient associé connu comme ayant le trouble neurologique ;
(d)(v) comparer (540) les sommes de bonne santé et les sommes de trouble; et
(d)(vi) décider (545) de la présence probable du trouble neurologique si l'une quelconque des sommes de trouble est supérieure à l'une quelconque des sommes de bonne santé.

12. Procédé selon l'une quelconque des revendications 1 à 7, dans lequel l'identification concerne la possibilité de traitement, par un premier régime de traitement, du patient sous évaluation.

13. Procédé selon la revendication 12, dans lequel l'étape (c) inclut la comparaison (625) de chaque projection à au moins un vecteur de signal ERP de caractérisation d'au moins un patient, respectivement, connu comme pouvant être traité par le premier régime de traitement.

14. Procédé selon la revendication 12, dans lequel l'étape (d) comporte les étapes consistant à :
(d)(i) pour chaque projection et chacun des vecteurs de signal ERP de caractérisation de chacun des patients, respectivement, connus comme pouvant être traités par le premier régime de traitement, dériver une première valeur de pondération qui est déterminée par un degré de corrélation entre la projection et le vecteur de signal ERP de caractérisation du patient connu comme pouvant être traité par le premier régime de traitement,
(d)(ii) pour chaque vecteur de signal ERP de caractérisation d'un patient connu comme pouvant être traité par le premier régime de traitement, additionner les premières valeurs de pondération pour toutes les projections pour produire une première somme ; et
(d)(ii) décider de la probable sensibilité du patient sous évaluation au premier régime de traitement, sur la base des premières sommes.

15. Procédé selon la revendication 14, dans lequel ladite étape (d) comporte de plus les étapes consistant à :
(d)(iv) pour chaque projection et chacun des vecteurs de signal ERP de caractérisation de chacun des patients, respectivement, connus comme pouvant être traités par un second régime de traitement, dériver une seconde valeur de pondération qui est déterminée par un degré de corrélation entre la projection et le vecteur de signal ERP de caractérisation du patient connu comme pouvant être traité par le second régime de traitement ;
(d)(v) pour chaque vecteur de signal ERP de caractérisation d'un patient connu comme pouvant être traité par le second régime de traitement, additionner les secondes valeurs de pondération pour toutes les projections pour produire une seconde somme;
(d)(vi) comparer les premières sommes et les secondes sommes, et
(d)(vii) décider de la probable sensibilité du patient sous évaluation au premier régime de traitement, relativement à la probable sensibilité au second régime de traitement, sur la base des premières et secondes sommes.

16. Procédé selon l'une quelconque des revendications 1 à 7, dans lequel l'identification concerne l'état actuel du patient sous évaluation, comme indiqué par les projections, après le début d'un traitement du trouble neurologique.

17. Procédé selon la revendication 16, dans lequel l'étape (c) inclut la comparaison (725) de chaque projection à au moins un vecteur de signal ERP de caractérisation d'au moins un patient, respectivement, ayant un état neurologique connu.

18. Procédé selon la revendication 17, dans lequel l'étape (c) inclut la comparaison de chaque projection à au moins un vecteur de signal ERP de caractérisation d'au moins un patient, respectivement, qui est neurologiquement en bonne santé.

19. Procédé selon la revendication 17, dans lequel l'étape (c) inclut la comparaison de chaque projection à au moins un vecteur de signal ERP de caractérisation d'au moins un patient, respectivement, qui est affecté par le trouble neurologique du patient en cours de traitement.

20. Procédé selon la revendication 17, dans lequel l'étape (d) inclut les étapes consistant à :
(d)(i) pour chacun des vecteurs de signal ERP de caractérisation d'au moins un patient, respectivement, et pour chaque projection, dériver une valeur de pondération de progrès, où la valeur de pondération de progrès est déterminée par un degré de corrélation entre la projection et le vecteur de signal ERP de caractérisation d'un patient ayant un état neurologique connu ;
(d)(ii) pour chacun des vecteurs de signal ERP de caractérisation d'au moins un patient, respectivement, additionner les valeurs de pondération de progrès pour toutes les projections, pour produire une somme de progrès ; et
(d)(iii) fournir une indication concernant l'état actuel probable du patient en cours de traitement, sur la base des sommes de progrès.

21. Procédé selon l'une quelconque des revendications 1 à 7, dans lequel l'identification concerne la nature et l'étendue des effets secondaires subis par le patient en réponse à un régime de traitement contre un trouble neurologique.

22. Procédé selon la revendication 21, dans lequel l'étape (d) comporte la comparaison (825) de chaque projection à au moins un vecteur de signal ERP de caractérisation d'au moins un patient, respectivement, ayant au moins un état neurologique connu, respectivement.

23. Procédé selon la revendication 22, dans lequel l'étape (d) comporte les étapes consistant à :
(d)(i) pour chaque vecteur de signal ERP de caractérisation d'un patient ayant un état neurologique connu et pour chaque projection, dériver une valeur de pondération d'effet secondaire, où chaque valeur de pondération d'effet secondaire est déterminée par le degré de corrélation entre la projection et le vecteur de signal ERP de caractérisation du patient ayant un état neurologique connu ;
(d)(ii) pour chaque vecteur de signal ERP de caractérisation d'un patient ayant un état neurologique connu, additionner les valeurs de pondération d'effet secondaire pour toutes les projections pour produire une somme d'effet secondaire ; et
(d)(iii) fournir une indication de la nature et de l'étendue probables des effets secondaires, sur la base des sommes d'effet secondaire.

24. Procédé selon l'une quelconque des revendications 1 à 7, dans lequel l'identification concerne les résultats d'un régime de traitement pour un trouble neurologique.

25. Procédé selon la revendication 24, dans lequel l'étape (c) inclut la comparaison (925) de chaque projection à au moins un vecteur de signal ERP de caractérisation d'au moins un patient, respectivement, dans lequel le ou chaque patient présente le ou chaque état neurologique connu, respectivement.

26. Procédé selon la revendication 25, dans lequel l'étape (d) comporte les étapes consistant à :
(d)(i) pour chaque vecteur de signal ERP de caractérisation d'un patient ayant un état neurologique connu et pour chaque projection, dériver une valeur de pondération de comparaison, où chaque valeur de pondération de comparaison est déterminée par le degré de corrélation entre la projection et le vecteur de signal ERP de caractérisation du patient ayant un état neurologique connu ;
(d)(ii) pour chaque vecteur de signal ERP de caractérisation d'un patient ayant un état neurologique connu, additionner les valeurs de pondération de comparaison pour toutes les projections pour produire une somme de comparaison ; et
(d)(iii) fournir une indication caractérisant les effets du régime de traitement, sur la base des sommes de comparaison.

27. Programme informatique incluant des étapes de programme informatique conçues pour exécuter le procédé selon l'une quelconque des revendications précédentes lorsqu'il est exécuté par un ordinateur conçu de manière adaptée (1100).

28. Support lisible par ordinateur (1118, 1122, 1112, 1128) exécutant le programme informatique de la revendication 27.

29. Appareil pour fournir une identification se rapportant à un trouble neurologique, comportant:
un appareil d'essai de potentiel de réponse évoqué (ERP) pour réaliser plusieurs essais ERP sur un patient sous évaluation ;
des moyens pour générer (1015) plusieurs projections de données ERP dérivées des essais ERP, en éliminant des composantes du vecteur ; **caractérisé par**
des moyens pour comparer (1020) chaque projection à des projections de données ERP de caractérisation d'autres patients, pour produire des résultats de comparaison pour chaque projection ; et
des moyens pour déterminer (1025) une identification concernant le trouble neurologique par un traitement de vote pondéré qui est basé sur les résultats de comparaison.

30. Appareil selon la revendication 29, dans lequel les moyens d'exécution sont conçus pour répéter un essai ERP approximativement une fois toutes les une à deux secondes, pendant approximativement deux minutes.

31. Appareil selon la revendication 29, dans lequel les données ERP comportent des signaux ERP obtenus dans les essais réalisés sur le patient sous évaluation, l'appareil comportant de plus:
des moyens pour caractériser (115) les signaux ERP (400) pour produire un vecteur de signal ERP de caractérisation (410A, 410B, 410C) pour le patient sous évaluation ; et
dans lequel les moyens de génération sont conçus pour générer les projections à partir du vecteur de signal ERP de caractérisation du patient sous évaluation.

32. Appareil selon la revendication 31, dans lequel les moyens de caractérisation sont conçus pour concaténer (215), pour chaque essai ERP, les signaux (305, 310, 315) reçus par des électrodes d'un appareil d'essai ERP dans un ordre prédéterminé pour former un signal concaténé (320), pour échantillonner (220), pour chaque essai ERP, le signal concaténé (320) à une fréquence d'échantillonnage prédéterminée, pour former un vecteur de valeurs d'amplitude (410A, 410B, 410C) pour chaque essai ERP, et pour établir la moyenne (230) des valeurs d'amplitude correspondantes prises dans chaque vecteur, pour former le vecteur de signal ERP de caractérisation du patient sous évaluation.

33. Appareil selon la revendication 32, dans lequel la fréquence d'échantillonnage est approximativement d'une fois toutes les 10 millisecondes.

34. Appareil selon l'une quelconque des revendications 31 à 33, dans lequel les moyens de génération sont conçus de manière aléatoire pour supprimer des composantes du vecteur de signal ERP de caractérisation du patient sous évaluation pour créer les projections.

35. Appareil selon l'une quelconque des revendications 31 à 33, dans lequel les moyens de génération sont conçus pour supprimer des composantes du vecteur de signal ERP de caractérisation du patient sous évaluation conformément à un algorithme déterministe pour créer les projections.

36. Appareil selon l'une quelconque des revendications 29 à 35, dans lequel l'identification concerne la présence du trouble neurologique chez le patient sous évaluation.

37. Appareil selon la revendication 36, dans lequel les moyens de comparaison sont conçus pour comparer (125) chaque projection à au moins un vecteur de signal ERP de caractérisation d'au moins un patient, respectivement, connu comme n'étant pas affecté par le trouble neurologique.

38. Appareil selon la revendication 36 ou la revendication 37, dans lequel les moyens de comparaison sont conçus pour comparer (130) chaque projection à au moins un vecteur de signal ERP de caractérisation d'au moins un patient, respectivement, connu comme étant affecté par le trouble neurologique.

39. Appareil selon la revendication 37 ou 38, dans lequel les moyens de détermination sont conçus pour :
dériver (515) une valeur de pondération de bonne santé pour chaque projection et chaque vecteur de signal ERP de caractérisation d'un patient en bonne santé, où la valeur de pondération de bonne santé est déterminée par un degré de corrélation entre la projection et le vecteur de signal ERP de caractérisation du patient en bonne santé, de sorte qu'un plus grand degré de corrélation entre la projection et le vecteur de signal ERP de caractérisation du patient en bonne santé correspond à une plus grande valeur de pondération de bonne santé;
dériver (520) une valeur de pondération de trouble pour chaque projection et chaque vecteur de signal ERP de caractérisation d'un patient connu comme ayant le trouble neurologique, où la valeur de pondération de trouble est déterminée par un degré de corrélation entre la projection et le vecteur de signal ERP de caractérisation du patient connu comme ayant le trouble neurologique, de sorte qu'un plus grand degré de corrélation entre la projection et le vecteur de signal ERP de caractérisation du patient comme ayant le trouble neurologique correspond à une plus grande valeur de pondération de trouble;
pour chaque vecteur de signal ERP de caractérisation d'un patient en bonne santé, additionner (530) les valeurs de pondération de bonne santé pour toutes les projections, afin de produire une somme de bonne santé correspondant au patient en bonne santé associé ;
pour chaque vecteur de signal ERP de caractérisation d'un patient connu comme ayant un trouble neurologique, additionner (535) les valeurs de pondération de trouble pour toutes les projections, pour créer une somme de trouble correspondant au patient associé connu pour le fait d'avoir le trouble neurologique ;
comparer les sommes de bonne santé et les sommes de trouble ; et
décider de la présence probable du trouble neurologique sur la base du fait que l'une quelconque des sommes de trouble est supérieure à l'une quelconque des sommes de bonne santé.

40. Appareil selon l'une quelconque des revendications 29 à 35, dans lequel l'identification concerne la possibilité de traitement, quant à un premier régime de traitement, du patient sous évaluation.

41. Appareil selon la revendication 40, dans lequel les moyens de comparaison sont conçus pour comparer (625) chaque projection à au moins un vecteur de signal ERP de caractérisation d'au moins un patient, respectivement, connu comme pouvant être traité par le premier régime de traitement.

42. Appareil selon la revendication 40, dans lequel les moyens de détermination sont conçus pour :
pour chaque projection et chacun des vecteurs de signal ERP de caractérisation de chacun des patients, respectivement, connus comme pouvant être traités par le premier régime de traitement, dériver une première valeur de pondération qui est déterminée par un degré de corrélation entre la projection et le vecteur de signal ERP de caractérisation du patient connu comme pouvant être traité par le premier régime de traitement,
pour chaque vecteur de signal ERP de caractérisation d'un patient connu comme pouvant être traité par le premier régime de traitement, additionner les premières valeurs de pondération pour toutes les projections pour produire une première somme ; et
décider de la probable sensibilité du patient sous évaluation au premier régime de traitement, sur la base des premières sommes.

43. Appareil selon la revendication 42, dans lequel les moyens de détermination sont de plus conçus pour :
pour chaque projection et chacun des vecteurs de signal ERP de caractérisation de chacun des patients, respectivement, connus comme pouvant être traités par un second régime de traitement, dériver une seconde valeur de pondération qui est déterminée par un degré de corrélation entre la projection et le vecteur de signal ERP de caractérisation du patient connu comme pouvant être traité par le second régime de traitement ;
pour chaque vecteur de signal ERP de caractérisation d'un patient connu comme pouvant être traité par le second régime de traitement, additionner les secondes valeurs de pondération pour toutes les projections pour produire une seconde somme;
comparer les premières sommes et les secondes sommes, et
décider de la probable sensibilité du patient sous évaluation au premier régime de traitement, relativement à la probable sensibilité au second régime de traitement, sur la base des premières et secondes sommes.

44. Appareil selon l'une quelconque des revendications 29 à 35, dans lequel l'identification concerne l'état actuel du patient sous évaluation, comme indiqué par les projections, après le début d'un traitement du trouble neurologique.

45. Appareil selon la revendication 44, dans lequel les moyens de comparaison sont conçus pour comparer (725) chaque projection à au moins un vecteur de signal ERP de caractérisation d'au moins un patient, respectivement, ayant un état neurologique connu.

46. Appareil selon la revendication 45, dans lequel les moyens de comparaison sont conçus pour comparer chaque projection à au moins un vecteur de signal ERP de caractérisation d'au moins un patient, respectivement, qui est neurologiquement en bonne santé.

47. Appareil selon la revendication 45, dans lequel les moyens de comparaison sont conçus pour comparer chaque projection à au moins un vecteur de signal ERP de caractérisation d'au moins un patient, respectivement, qui est affecté par le trouble neurologique du patient en cours de traitement.

48. Appareil selon la revendication 45, dans lequel les moyens de détermination sont conçus pour :
pour chacun des vecteurs de signal ERP de caractérisation d'au moins un patient, respectivement, et pour chaque projection, dériver une valeur de pondération de progrès, où la valeur de pondération de progrès est déterminée par un degré de corrélation entre la projection et le vecteur de signal ERP de caractérisation d'un patient ayant un état neurologique connu ;
pour chacun des vecteurs de signal ERP de caractérisation d'au moins un patient, respectivement, additionner les valeurs de pondération de progrès pour toutes les projections, pour produire une somme de progrès ; et
délivrer une indication sur l'état actuel probable du patient en cours de traitement, sur la base des sommes de progrès.

49. Appareil selon l'une quelconque des revendications 29 à 35, dans lequel l'identification concerne la nature et l'étendue des effets secondaires subis par le patient en réponse à un régime de traitement contre un trouble neurologique.

50. Appareil selon la revendication 49, dans lequel les moyens de comparaison sont conçus pour comparer (825) chaque projection à au moins un vecteur de signal ERP de caractérisation d'au moins un patient, respectivement, ayant au moins un état neurologique connu, respectivement.

51. Appareil selon la revendication 50, dans lequel les moyens de détermination sont conçus pour :
pour chaque vecteur de signal ERP de caractérisation d'un patient ayant un état neurologique connu et pour chaque projection, dériver une valeur de pondération d'effet secondaire, où chaque valeur de pondération d'effet secondaire est déterminée par le degré de corrélation entre la projection et le vecteur de signal ERP de caractérisation du patient ayant un état neurologique connu ;
pour chaque vecteur de signal ERP de caractérisation d'un patient ayant un état neurologique connu, additionner les valeurs de pondération d'effet secondaire pour toutes les projections pour produire une somme d'effet secondaire ; et
fournir une indication de la nature et de l'étendue probables des effets secondaires, d'après les sommes d'effet secondaire.

52. Appareil selon l'une quelconque des revendications 29 à 35, dans lequel l'identification concerne les résultats d'un régime de traitement contre un trouble neurologique.

53. Appareil selon la revendication 52, dans lequel les moyens de comparaison sont conçus pour comparer chaque projection à au moins un vecteur de signal ERP de caractérisation d'au moins un patient, respectivement, dans lequel le ou chaque patient présente le ou chaque état neurologique connu, respectivement.

54. Appareil selon la revendication 53, dans lequel les moyens de détermination sont conçus pour :
pour chaque vecteur de signal ERP de caractérisation d'un patient ayant un état neurologique connu et pour chaque projection, dériver une valeur de pondération de comparaison, où chaque valeur de pondération de comparaison est déterminée par le degré de corrélation entre la projection et le vecteur de signal ERP de caractérisation du patient ayant un état neurologique connu ;
pour chaque vecteur de signal ERP de caractérisation d'un patient ayant un état neurologique connu, additionner les valeurs de pondération de comparaison pour toutes les projections pour produire une somme de comparaison ; et
délivrer une indication caractérisant les effets du régime de traitement, sur la base des sommes de comparaison.
